# EUROPEAN PATENT APPLICATION

(11) **EP 4 480 486 A1**
(43) Date of publication of application: **25.12.2024**
(21) Application number: 23752989.6
(22) Date of filing: 14.02.2023
(51) Int. Cl.: A61K 31/7032, A61K 9/127, A61K 39/00, A61P 31/00, A61P 35/00, A61P 37/04

(54) **NKT CELL LIGAND-CONTAINING LIPOSOME COMPOSITION**

(30) Priority: 14.02.2022 JP 2022020306
(71) Applicant: RIKEN, Wako-shi, Saitama 351-0198 (JP)
(72) Inventor: TANIGUCHI, Masaru, (deceased) (JP); OHNO, Hiroshi, Wako-shi, Saitama 351-0198 (JP); TAIDA, Takashi, Wako-shi, Saitama 351-0198 (JP); JINNOHARA, Toshi, Wako-shi, Saitama 351-0198 (JP); TAKAHASHI, Masumi, Wako-shi, Saitama 351-0198 (JP); MORIYAMA, Rui, Tokyo 105-0022 (JP); HASHIMOTO, Kouichi, Tokyo 105-0022 (JP); SASAKI, Tomoki, Tokyo 105-0022 (JP); NAKANO, Yuta, Tokyo 105-0022 (JP); AMARI, Hirokuni, Tokyo 113-0034 (JP); KIKUCHI, Hiroshi, Tokyo 113-0034 (JP)
(74) Representative: Vossius & Partner Patentanwälte Rechtsanwälte mbB
(86) International application number: PCT/JP2023/005048
(87) International publication number: WO 2023/153527

(57) **Abstract**

The present invention aims to provide an NKT cell ligand-containing preparation that can artificially activate NKT cells in a living body and is useful as an anti-malignant tumor agent and the like.

A liposome composition containing a glycolipid selected from the group consisting of a compound represented by the formula (I), a compound represented by the formula (II), and salts thereof:
the formula (I): 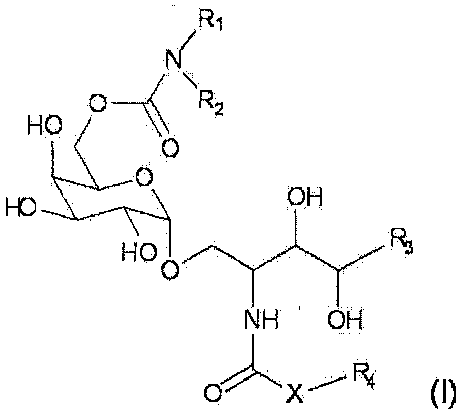 wherein each symbol is as defined in the specification,
the formula (II): 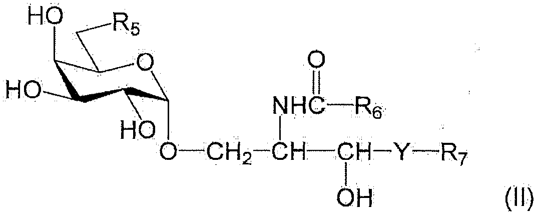 wherein each symbol is as defined in the DESCRIPTION.

## Description

### [Technical Field]

The present invention relates to a liposome composition containing an NKT cell ligand (glycolipid described later) that is useful as an anti-malignant tumor agent, an anti-infective agent, an NKT cell activator, an IFN-γ production inducer, an activator for innate immune system lymphocytes and acquired immune system lymphocytes, and a long-term immune memory inducer.

### [Background Art]

Natural killer (hereinafter NK) T cells are immunocytes belonging to a new lymphocyte lineage that exhibit characteristics different from those of other lymphocyte lineages (T, B, and NK cells). NKT cells are related to NK cells because cytotoxic perforin granules are present therein (non-patent document 1). However, because NKT cells express not only NK cell markers, but also T cell receptors (TCRs), they have been shown to represent a new class of cells that are completely different (non-patent document 2). NKT cells can produce both Th1 type cytokine [mainly interferon (IFN)-γ] produced by T helper(Th)1 cell that promotes immunostimulatory action, and Th2 type cytokine [mainly interleukin (IL)-4, IL-10, and the like] produced by Th2 cell that promotes immunosuppressive action (non-patent document 3). In other words, NKT cells can induce both activation and quieting of the immune system, which suggests the possible role of the immune system in the balance adjustment (non-patent document 4). Therefore, when the function of NKT cells can be controlled, various diseases, particularly cancer and infectious disease, caused by abnormal balance of the immune system can be treated.

The characteristic of NKT cells that is attracting the greatest attention resides in the fact that the antigen receptor α chain expressed in NKT cells is the same in all the individuals belonging to one certain species. This essentially shows that all NKT cells of the same species of organism are activated by recognizing the same substance. As such, the α chain is Vα24Jα18 for humans and Vα14Jα18 for mice, there is a very high homology between the two species. For the β chain, which forms a pair with the α chain, only a very limited number of kinds are known, and therefore, this NKT cell antigen receptor is called "invariant TCR" and cannot be expressed by normal T cells. It is also characteristic that the antigen receptor (TCR) of NKT cells recognizes glycolipid, whereas the antigen receptor of T cells recognizes a protein fragment.

A wide variety of sphingoglycolipids are known to exist in living organisms. In general sphingoglycolipids in the living organisms, various sugars or sugar chains are bound to ceramides via β-bonds, and they are present in the cellular membranes of various organs.

Meanwhile, it is known that sphingoglycolipids comprising sugars bound to ceramides via α-bonds possess potent immunostimulatory action and antitumor activity, α-Galactosylceramides, typified by agelasphins, are glycolipids isolated from extracts from Agelas mauritianus, a kind of marine sponge, and have been reported to potently activate NKT cells (non-patent document 5). α-Galactosyl ceramides are taken by antigen-presenting cells (APC) represented by dendritic cell (DC) and the like, and presented on a cellular membrane by CDld molecule similar to major histocompatibility complex (MHC) class I molecule. NKT cells are activated by recognizing a complex of the thus-presented CDld molecule and α-galactosylceramide by using an NKT cell-specific antigen receptor, whereby various immune reactions are initiated.

Heretofore, various analogs have been synthesized, and the correlation between the structure and the activity has been researched. It has been clarified that, among the series of synthesized analogs, KRN7000 (α-GalCer) developed by Kirin Brewery Co., Ltd. shows an extremely strong anti-tumor activity, and the corresponding β-form (β-GalCer) does not show an immunostimulatory activity (Non Patent Literature 6). KRN7000 is sphingoglycolipid containing a ceramide resulting from the acylation of the sphingosine base by a long-chain fatty acid, and galactose bound thereto in α-configuration.

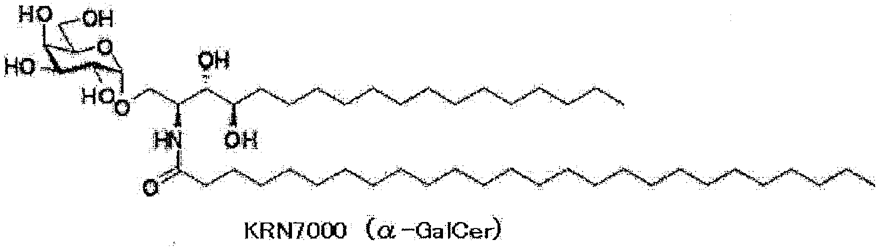

In recent years, with a focus on the above-described functions of NKT cells, a therapeutic drug of cancer, which contains KRN7000 as an active ingredient, has been developed. However, NKT cells activated by the administration of KRN7000 produce IFN-γ, which is a cytokine useful for the treatment of cancer and infectious diseases and inducing immunostimulatory activity, as well as simultaneously produce IL-4, which is a cytokine inducing an immunosuppressive action. As a result, the effects of the both are cancelled by each other, posing the problem of lack of sufficient effect of the treatment of cancer and infectious diseases.

For the treatment of cancer and infectious diseases, many α-GalCer derivatives have been developed that strongly activate NKT cells and preferentially produce IFN-γ over IL-4 (Patent Literatures 1, 2).

Patent Literature 1 describes a compound represented by the following formula or a salt thereof: 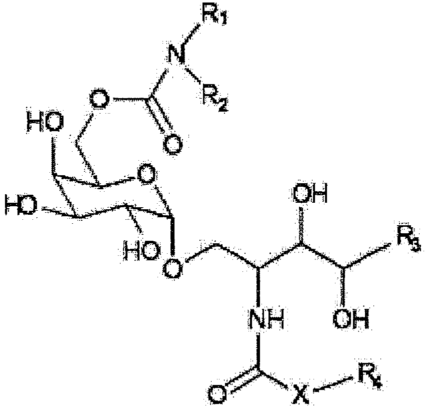 wherein
X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30.

Patent Literature 2 describes a compound represented by the following formula or a salt thereof: 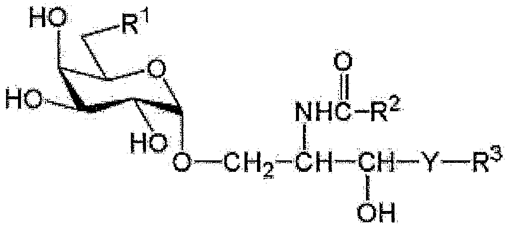 wherein R¹ is a hydrogen atom, an alkyl group having a carbon number of 1 to 7, an alkoxy group having a carbon number of 1 to 6 or a halogen atom, R² and R³ are each independently a substituted or unsubstituted hydrocarbon group having a carbon number of 1 to 28, and Y is -CH₂-, -CH(OH)- or -CH=CH-, provided that when R¹ is a hydrogen atom, then R² is a substituted or unsubstituted hydrocarbon group having a carbon number of 24 to 28.

NKT cell ligands such as α-GalCer and the like are glycolipids. Thus, when administered directly to a living body, the hydrophobic group forms micelles, which in turn prevent efficient delivery to the target cell and the effect could not be exerted. However, it was clarified that a potent antitumor effect is exerted by a cell therapy in which the ligands are administered through presentation by antigen-presenting cells.

Based on the above-mentioned studies, clinical trials of cancer treatment drugs have been conducted to artificially activate NKT cells in the living body by administering antigen-presenting cell (APC) preparations (cell preparations) pulsed with a glycolipid NKT cell ligand, and results with extremely high effectiveness have been obtained (Non Patent Literatures 7 to 9) .

However, even though the above-mentioned cell preparations are effective, various problems have been pointed out (risk of anaphylaxis due to allogenic cell preparations, complicated cell separation of autologous cells, instability of patient cell preparations due to freezing and thawing, and the like). In fact, the production process of cell preparations requires an extremely long time, complicated procedures, and high costs, as exemplified by cell separation and purification, cell differentiation culture, pulse culture, freezing and thawing of cell preparations, and removal of dead cell aggregates before administration. In addition, when an allogenic cell preparation is administered multiple times, there is a risk of developing anaphylaxis. Therefore, the development of an NKT cell ligand-containing preparation based on a new concept that does not use cells is desired.

Malignant tumors are cells that develop from self-tissues, and do not have, unlike pathogens, adjuvant substances that activate NKT cells. Therefore, they cannot activate NKT cells, which are essential for inducing long-term immune memory, and have difficulty in inducing and maintaining immune memory against malignant tumors. Accordingly, the development of a preparation that can induce and maintain immune memory against malignant tumors is desired.

### [Citation List]

### [Patent Literature]

[Patent Literature 1] WO 2013/162016
[Patent Literature 2] WO 2009/119692

### [Non Patent Literature]

[Non Patent Literature 1] Proc. Natl. Acad. Sci. USA 1998, 95, 5690-5693
[Non Patent Literature 2] J. Immunol. 1995, 155, 2972-2983
[Non Patent Literature 3] J. Immunol. 1998, 161, 3271-3281
[Non Patent Literature 4] Science, 1997, 278, 1623-1626
[Non Patent Literature 5] Science, 1997, 278, 1626-1629
[Non Patent Literature 6] J. Med. Chem. 1995, 38, 2176-2187
[Non Patent Literature 7] J Immunol 1999; 163: 2387-2391
[Non Patent Literature 8] Ishikawa et al., Clin Cancer Res. 11: 1910-1917, 2005, 269
[Non Patent Literature 9] Motohashi et al., J Immunol. 182:2492-501, 2009

### [Summary of Invention]

### [Technical Problem]

The present invention aims to provide an NKT cell ligand-containing preparation that can artificially activate NKT cells in a living body.

In addition, the present invention aims to provide an NKT cell ligand-containing preparation that induces and maintains long-term immune memory against malignant tumors by artificial activation of NKT cells in a malignant tumor-bearing living body and can control the progression, recurrence, and metastasis of malignant tumors by sustained attack on malignant tumors through immune memory.

Furthermore, the present invention aims to provide an NKT cell ligand-containing preparation that also induces and maintains long-term immune memory against pathogens in infectious diseases and can treat or prevent infectious diseases as an adjuvant agent.

### [Solution to Problem]

The present inventors have conducted intensive studies of the above-mentioned problems and found that the below-mentioned glycolipid, which is an NKT cell ligand, can be efficiently delivered to antigen-presenting cells in a living body by incorporating the glycolipid in a liposome and administering same to the body, as a result of which NKT cells are artificially activated, immune cells of the innate immune system and acquired immune system are activated and proliferated by IFN-γ produced by the activated NKT cells, and long-term immune memory can be induced. Based on such findings, the inventors have conducted further studies and completed the present invention. Accordingly, the present invention provides the following.
[1] A liposome composition comprising a glycolipid selected from the group consisting of a compound represented by the formula (I), a compound represented by the formula (II), and salts thereof (NKT cell ligand): the formula (I): 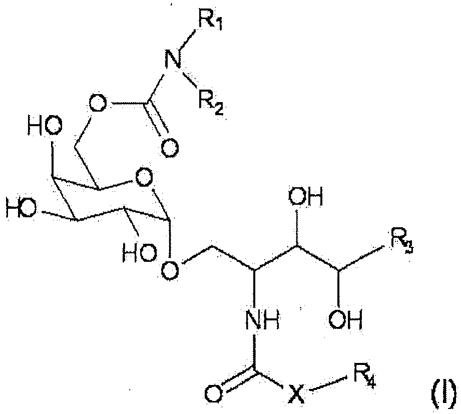
   wherein X is an alkylene group or -NH-;
   R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
   R₃ is a hydrocarbon group having a carbon number of 1 to 20; and
   R₄ is a hydrocarbon group having a carbon number of 1 to 30; the formula (II): 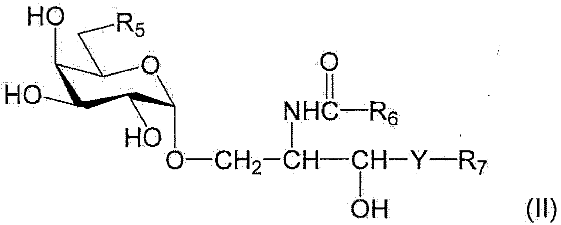
   wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.
[2] The composition of the above-mentioned [1], wherein the aforementioned glycolipid is a compound represented by the formula (I) or a salt thereof.
[3] The composition of the above-mentioned [2], wherein the aforementioned glycolipid is a compound represented by the following formula: 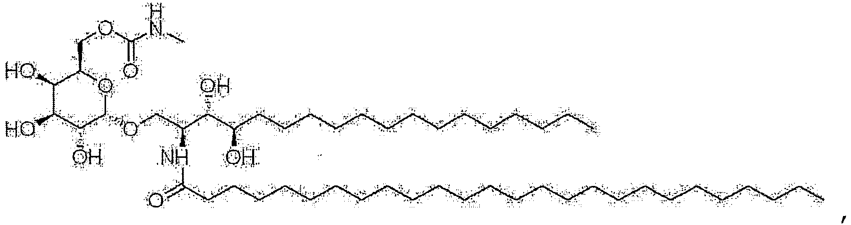 or a salt thereof.
[4] The composition of any of the above-mentioned [1] to [3], which is an anti-malignant tumor agent or an anti-infective agent.
[5] The composition of any of the above-mentioned [1] to [3], which is an NKT cell activator.
[6] The composition of any of the above-mentioned [1] to [3], which is an IFN-γ production inducer.
[7] The composition of any of the above-mentioned [1] to [3], which is an activator for innate immune system lymphocyte and acquired immune system lymphocyte.
[8] The composition of any of the above-mentioned [1] to [3], which is a long-term immune memory inducer.
[9] The composition of any of the above-mentioned [1] to [3], for delivering the aforementioned glycolipid to an antigen-presenting cell.
[10] A method for delivering a glycolipid (NKT cell ligand) selected from the group consisting of a compound represented by the formula (I), a compound represented by the formula (II), and salts thereof to an antigen-presenting cell, comprising administering a liposome composition comprising the glycolipid to a target: the formula (I): 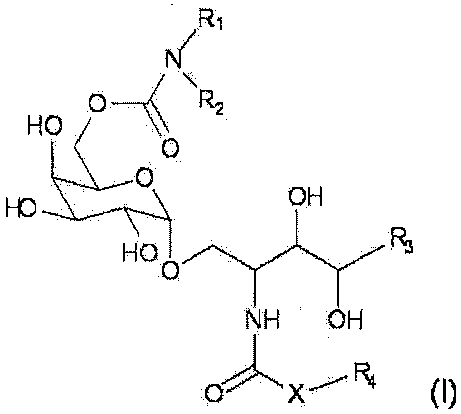
   wherein X is an alkylene group or -NH-;
   R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
   R₃ is a hydrocarbon group having a carbon number of 1 to 20; and
   R₄ is a hydrocarbon group having a carbon number of 1 to 30; the formula (II): 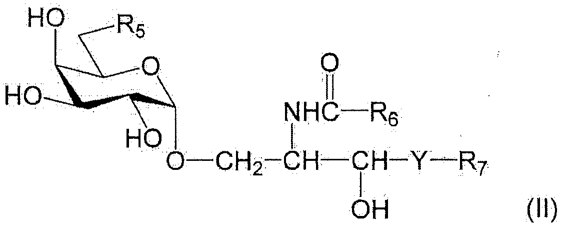 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.
[11] A method for treating a malignant tumor or an infectious disease, comprising administering a liposome composition comprising a glycolipid selected from the group consisting of a compound represented by the formula (I), a compound represented by the formula (II), and salts thereof to a target: the formula (I): 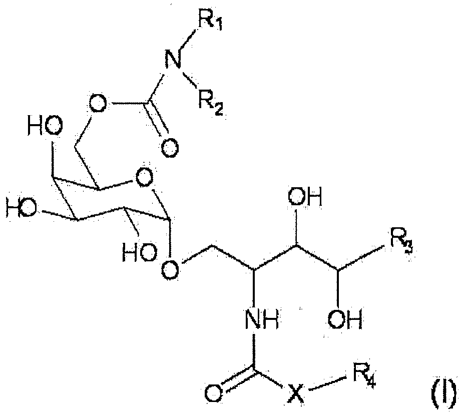
   wherein X is an alkylene group or -NH-;
   R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
   R₃ is a hydrocarbon group having a carbon number of 1 to 20; and
   R₄ is a hydrocarbon group having a carbon number of 1 to 30; the formula (II): 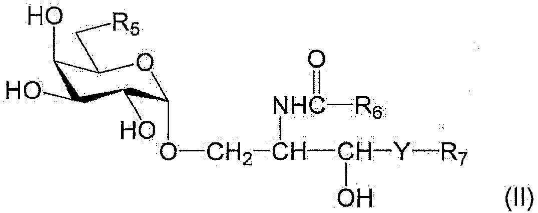 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.
[12] A method for activating NKT cells in a target, comprising administering a liposome composition comprising a glycolipid selected from the group consisting of a compound represented by the formula (I), a compound represented by the formula (II), and salts thereof to the target: the formula (I): 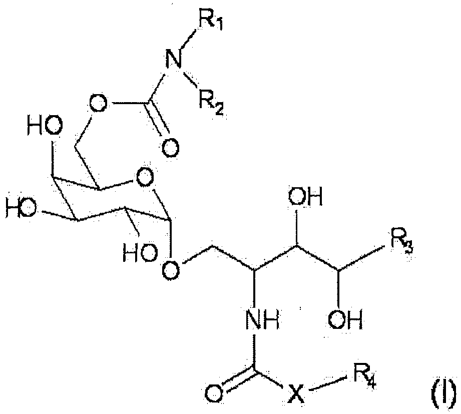
   wherein X is an alkylene group or -NH-;
   R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
   R₃ is a hydrocarbon group having a carbon number of 1 to 20; and
   R₄ is a hydrocarbon group having a carbon number of 1 to 30; the formula (II): 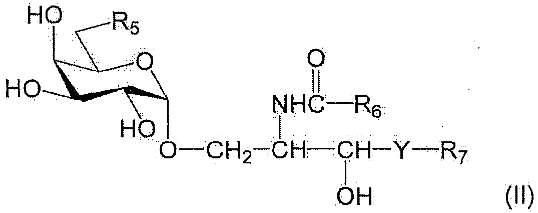 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.
[13] A method for inducing IFN-γ production in a target, comprising administering a liposome composition comprising a glycolipid selected from the group consisting of a compound represented by the formula (I), a compound represented by the formula (II), and salts thereof to the target: the formula (I): 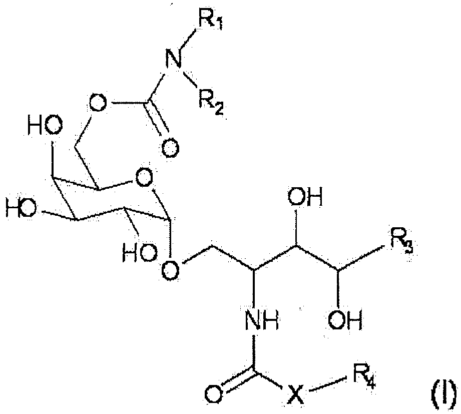
   wherein X is an alkylene group or -NH-;
   R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
   R₃ is a hydrocarbon group having a carbon number of 1 to 20; and
   R₄ is a hydrocarbon group having a carbon number of 1 to 30; the formula (II): 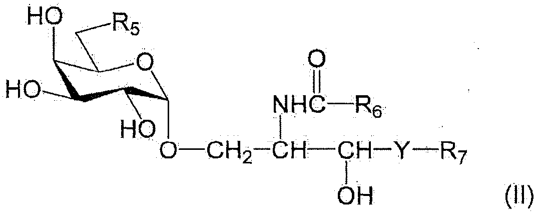 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.
[14] A method for activating innate immune system lymphocytes and acquired immune system lymphocytes in a target, comprising administering a liposome composition comprising a glycolipid selected from the group consisting of a compound represented by the formula (I), a compound represented by the formula (II), and salts thereof to the target: the formula (I): 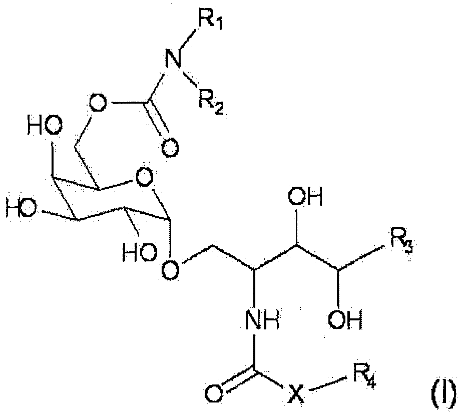
   wherein X is an alkylene group or -NH-;
   R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
   R₃ is a hydrocarbon group having a carbon number of 1 to 20; and
   R₄ is a hydrocarbon group having a carbon number of 1 to 30; the formula (II): 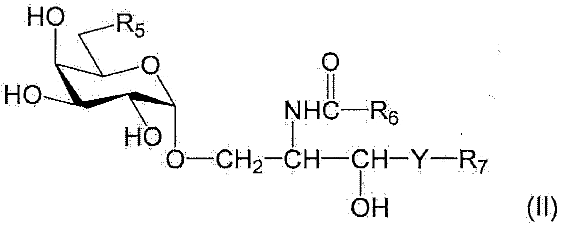 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.
[15] A method for inducing a long-term immune memory in a target, comprising administering a liposome composition comprising a glycolipid selected from the group consisting of a compound represented by the formula (I), a compound represented by the formula (II), and salts thereof to the target: the formula (I): 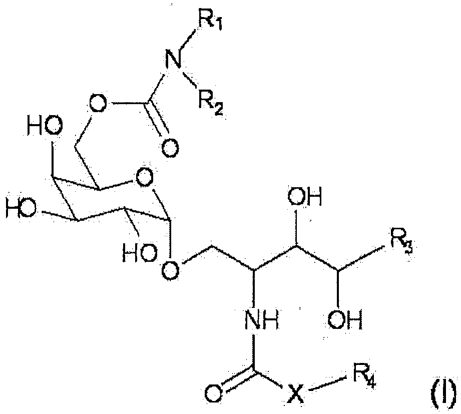
   wherein X is an alkylene group or -NH-;
   R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
   R₃ is a hydrocarbon group having a carbon number of 1 to 20; and
   R₄ is a hydrocarbon group having a carbon number of 1 to 30; the formula (II): 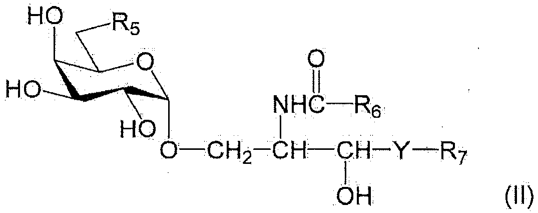 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.
[16] The composition of any of the above-mentioned [1] to [3], for use in delivering the aforementioned glycolipid to an antigen-presenting cell.
[17] The composition of any of the above-mentioned [1] to [3], for use in treating a malignant tumor or an infectious disease.
[18] The composition of any of the above-mentioned [1] to [3], for use in activating an NKT cell.
[19] The composition of any of the above-mentioned [1] to [3], for use in inducing IFN-γ production.
[20] The composition of any of the above-mentioned [1] to [3], for use in activating an innate immune system lymphocyte and an acquired immune system lymphocyte.
[21] The composition of any of the above-mentioned [1] to [3], for use in inducing a long-term immune memory.
[22] The composition of any of the above-mentioned [1] to [3], for use as a medicament.

### [Advantageous Effects of Invention]

The liposome composition of the present invention containing a glycolipid selected from the group consisting of a compound represented by the formula (I), a compound represented by the formula (II), and salts thereof (hereinafter also to be referred to as the liposome composition of the present invention) can be efficiently delivered to antigen-presenting cells in a living body by administering the composition to the body (particularly malignant tumor-bearing living body, or living body carrying infectious disease pathogen), as a result of which NKT cells are artificially activated, immune cells of the innate immune system and acquired immune system are activated and proliferated by IFN-γ produced by the activated NKT cells, and long-term immune memory can be induced.

Therefore, the liposome composition of the present invention is useful as an anti-malignant tumor agent, an anti-infective agent, an NKT cell activator, an IFN-γ production inducer, an innate immune system lymphocyte and an acquired immune system lymphocyte activator, and a long-term immune memory inducer.

Generally, there are two types of cancer cells of those that express antigens and those that have lost antigen expression. The liposome composition of the present invention can activate cell groups of both the innate immune system lymphocytes and the acquired immune system lymphocytes, so that the acquired immune system lymphocyte CD8 killer T cells attack the former and the innate immune system NK cells attack the latter, thus eliminating both cancer cells. Activation of innate immune system lymphocytes and acquired immune system lymphocytes in the patient's body can be expected to achieve an antitumor enhancing effect.

In addition, it has been reported that cancer cells that express mutant cancer antigens are newly born when tumor cells divide, and conventional treatment methods cannot deal with this. However, since the liposome composition of the present invention can also activate acquired immune system lymphocytes that correspond to mutant cancer cells in cancer tissues, mutant cancer cells can also be eliminated.

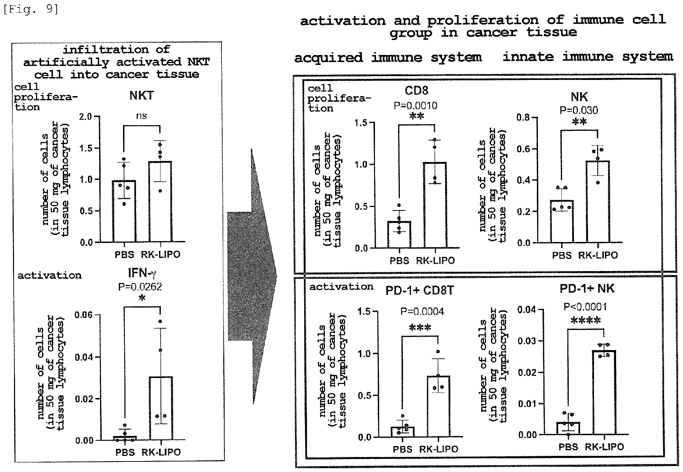

Based on the results of the Experimental Examples described later, the liposome composition of the present invention (NKT cell ligand-containing liposome) can be expected to achieve the following effects after administration to a living body.
1. By simply intravenously administering an extremely small amount of the NKT cell ligand (RK-163, RCAI-61, RCAI-137, etc.)-containing liposome of the present invention (1 ng of RK-163, RCAI-61, RCAI-137 per 25 g mouse; 2.4 mg of RK-163, RCAI-61, RCAI-137 when converted to amount for a human weighing 60 kg), NKT cells in vivo present outside the tumor tissue were artificially activated (see Experimental Example 5-1 described below). In fact, after administration of the NKT cell ligand (RK-163 or RCAI-61 or RCAI-137)-containing liposomes of the present invention, the number of artificially activated NKT cells increased and a large amount of IFN-γ, which is an adjuvant substance, was produced, whereby innate immune system · acquired immune system immune cell groups were strongly activated the cell proliferation was induced (see Experimental Examples 5-1, 5-2, 6, 7, 8, and 9 described below). In addition, it was confirmed that the NKT cell ligand (RK-163)-containing liposome of the present invention activates human NKT cells.
2. Artificially activated NKT cells strongly express the chemokine receptor CXCR6, and therefore efficiently infiltrate into cancer tissues by utilizing the density gradient of the chemokine CXCL16 produced by tumor tissues. NKT cells that have not been artificially activated also infiltrate into tumor tissues in the same manner, but the production of IFN-γ is not observed. It is clear therefrom that not only the presence of NKT cells but also artificial activation thereof are necessary (see the left diagram in Fig. 9 of Experimental Example 9 described later).
3. The artificially activated NKT cells that reached inside tumor tissues release large amounts of IFN-γ, which is an adjuvant substance, and strongly activate the innate immune system NK cells, acquired immune system CD8 killer T cells, CD4 helper T cells, and the like that have gathered in the cancer tissues to attack tumor cells, induce their proliferation, and enable them to attack tumor cells (see the right diagram in Fig. 9 of Experimental Example 9 described later).
4. At the same time, they induce immune memory cells of the CD8 killer T cell group, which are responsible for long-term immune memory and are the main body in attacking tumor cells (see Experimental Examples 10 and 11 described later). This enables sustained cancer attack. In fact, when the NKT cell ligand-containing liposome of the present invention was administered once in the presence of a cancer antigen, then left untreated, and a cancer cell transplantation experiment was performed one year later, the proliferation of transplanted cancer cells was extremely strongly suppressed, but the proliferation of cancer cells was not suppressed by liposomes that did not contain the NKT cell ligand or in individuals lacking NKT cells (NKT-KO mice) (see Experimental Example 12 described later). Therefrom it can be said that the suppression of malignant tumor proliferation by the NKT cell ligand-containing liposome of the present invention is the result of long-term immune memory induction dependent on the artificial activation of NKT cells.

The present invention relates to an "immune memory induction treatment technique for malignant tumors" that includes efficient artificial activation of NKT cells in a malignant tumor-bearing living body, followed by activation of immune cell groups and immune memory induction.

More specifically, the present invention relates to a method for producing an "NKT cell ligand-containing liposome" that artificially activates NKT cells in a malignant tumor-bearing living body, and a medicament for the treatment of malignant tumors that can control the progression, recurrence, and metastasis of all malignant tumors regardless of the type of malignant tumor by activating the immune system of a malignant tumor-bearing individual by inducing and maintaining long-term immune memory against malignant tumors, which has previously been difficult to induce and maintain in malignant tumor-bearing living bodies, by the administration of the liposome, and by inducing sustained anti-tumor activity through immune memory, and use of the medicament.

The present invention also relates to a medicament (vaccine or the like) that treats and prevents infectious diseases as an adjuvant agent, by inducing and maintaining long-term immune memory of pathogens in infectious diseases, and use of the medicament.

### [Brief Description of Drawings]

[Fig. 1]

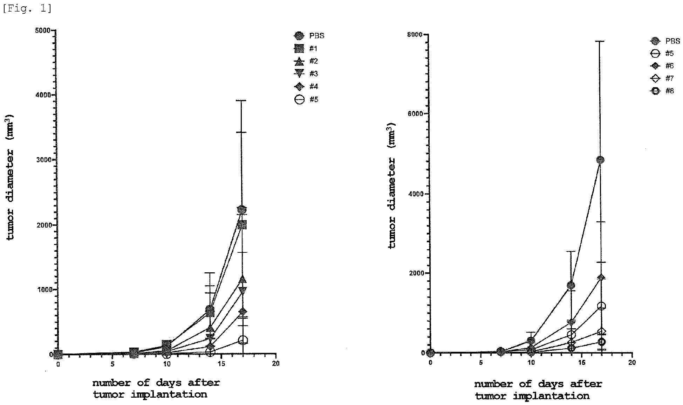

   Fig. 1 shows the results of Experimental Example 1-1 (left diagram) and Experimental Example 1-2 (right diagram). In the Figure, #1 shows RK-163-containing CAPTISOL solution, #2 to #8 show RK-163-containing liposome administration groups, and PBS shows PBS administration group (control).
[Fig. 2]

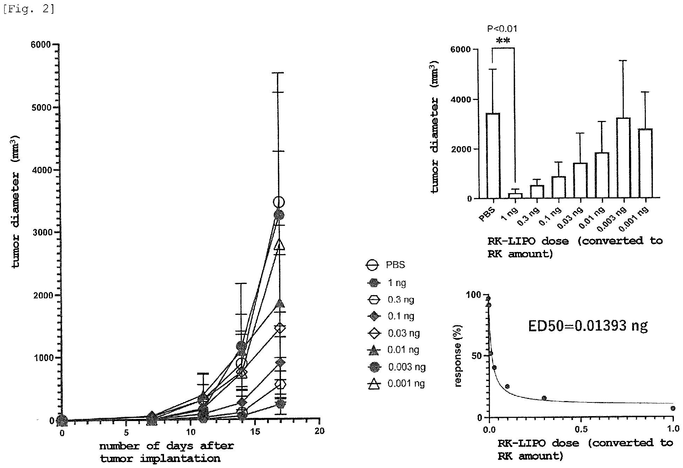

   Fig. 2 shows the results of Experimental Example 2. In the Figure, RK-LIPO shows RK-163-containing liposome: (#7) administration group, and PBS shows a PBS administration group (control). The RK-LIPO dose (converted to RK amount) in the right diagram, upper panel, was converted to a curve graph (right diagram, lower panel) and ED50 was calculated.
[Fig. 3]

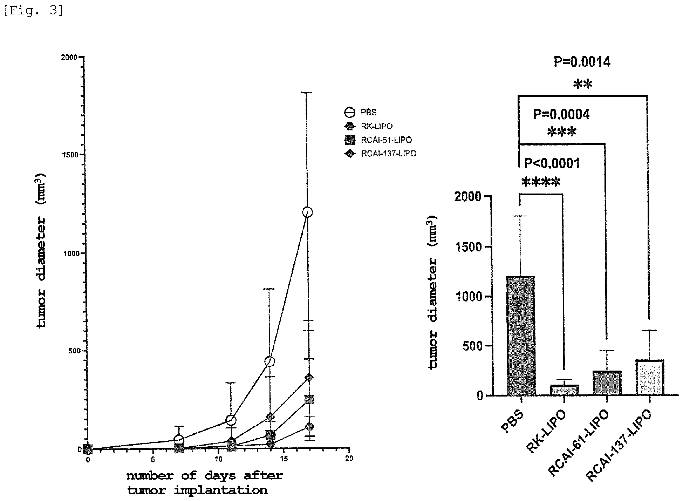

   Fig. 3 shows the results of Experimental Example 3. In the Figure, RK-LIPO shows RK-163-containing liposome: (#7) administration group, RCAI-61-LIPO shows RCAI-61-containing liposome: (#9) administration group, and RCAI-137-LIPO shows RCAI-137-containing liposome: (#10) administration group. PBS shows a PBS administration group (control).
[Fig. 4]

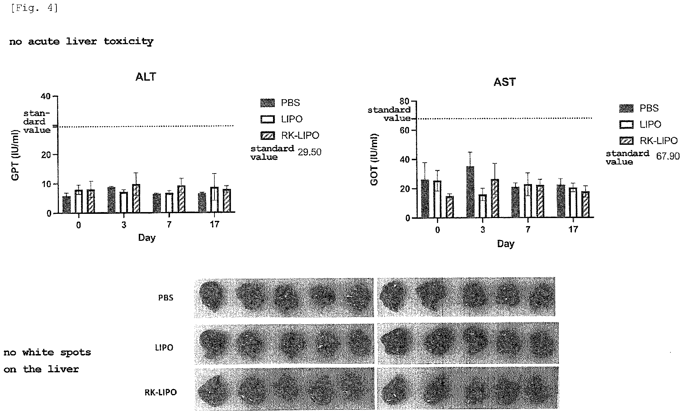

   Fig. 4 shows the results of Experimental Example 4. In the Figure, RK-LIPO shows RK-163-containing liposome: (#7) administration group, LIPO shows a liposome administration group not containing RK-163 (control), and PBS shows a PBS administration group (control).
[Fig. 5-1]

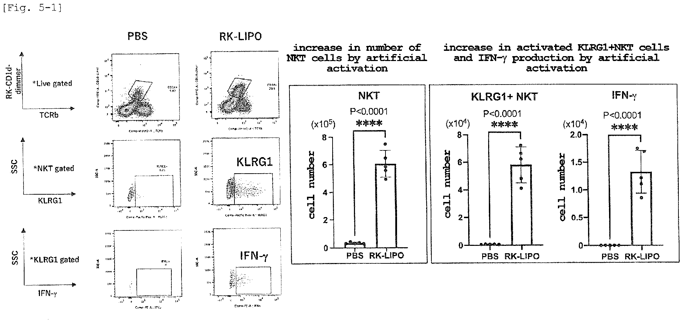

   Fig. 5-1 shows the results of Experimental Example 5-1. In the Figure, RK-LIPO shows RK-163-containing liposome: (#7) administration group, and PBS shows a PBS administration group (control).
[Fig. 5-2]

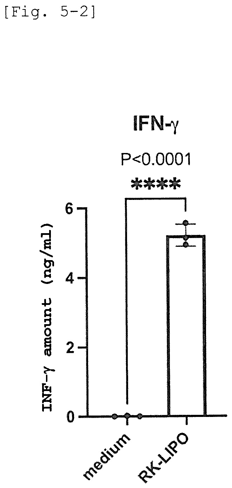

   Fig. 5-2 shows the results of Experimental Example 5-2. In the Figure, RK-LIPO shows RK-163-containing liposome: (#7) administration group, and medium shows a culture medium (medium) administration group (control).
[Fig. 6]

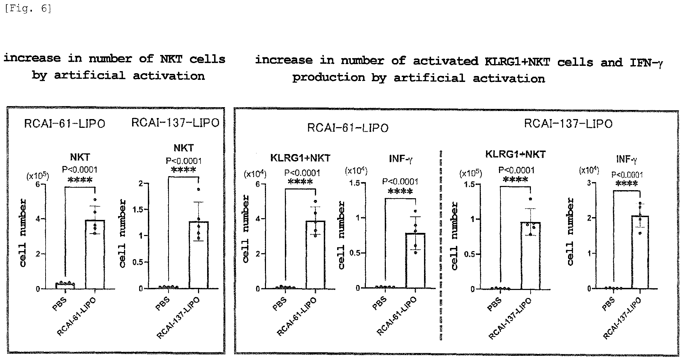

   Fig. 6 shows the results of Experimental Example 6. In the Figure, RCAI-61-LIPO shows RCAI-61-containing liposome: (#9) administration group, RCAI-137-LIPO shows RCAI-137-containing liposome: (#10) administration group, and PBS shows a PBS administration group (control).
[Fig. 7]

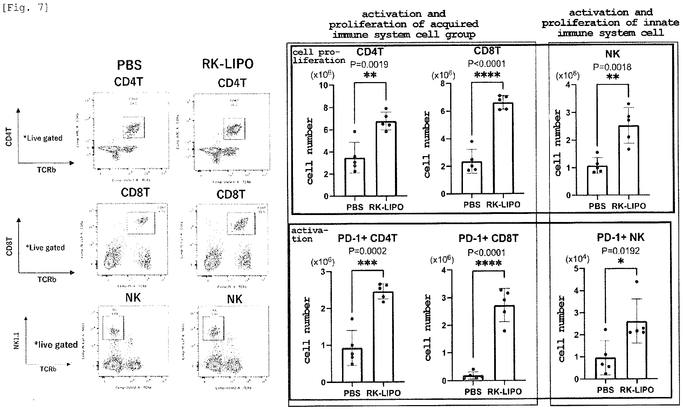

   Fig. 7 shows the results of Experimental Example 7. In the Figure, RK-LIPO shows RK-163-containing liposome: (#7) administration group, and PBS shows a PBS administration group (control).
[Fig. 8]

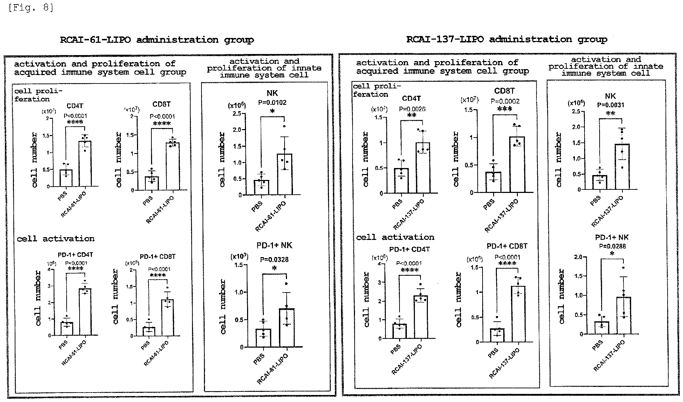

   Fig. 8 shows the results of Experimental Example 8. In the Figure, RCAI-61-LIPO in the left shows RCAI-61-containing liposome: (#9) administration group, PBS in the right shows a PBS administration group (control). In the Figure, RCAI-137-LIPO in the right shows RCAI-137-containing liposome: (#10) administration group, and PBS shows a PBS administration group (control).
[Fig. 9]
   Fig. 9 shows the results of Experimental Example 9. In the Figure, RK-LIPO shows RK-163-containing liposome: (#7) administration group, and PBS shows a PBS administration group (control).
[Fig. 10]

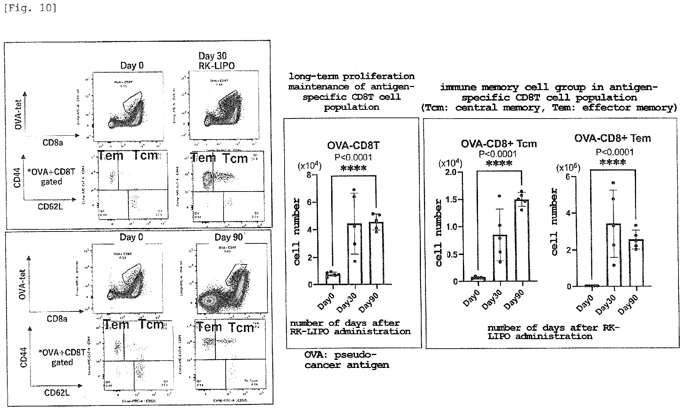

   Fig. 10 shows the results of Experimental Example 10. In the Figure, RK-LIPO shows RK-163-containing liposome: (#7) administration group. OVA shows a pseudo-cancer antigen.
[Fig. 11]

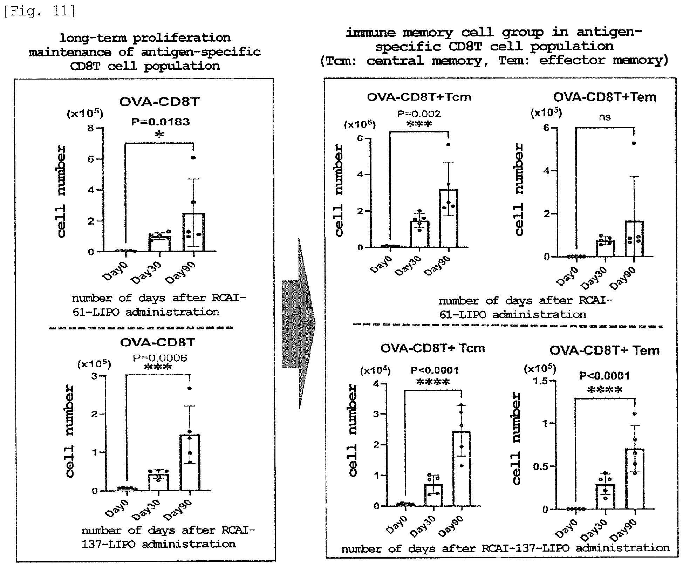

   Fig. 11 shows the results of Experimental Example 11. In the Figure, RCAI-61-LIPO shows RCAI-61-containing liposome: (#9) administration group, and RCAI-137-LIPO shows RCAI-137-containing liposome: (#10) administration group.
[Fig. 12]

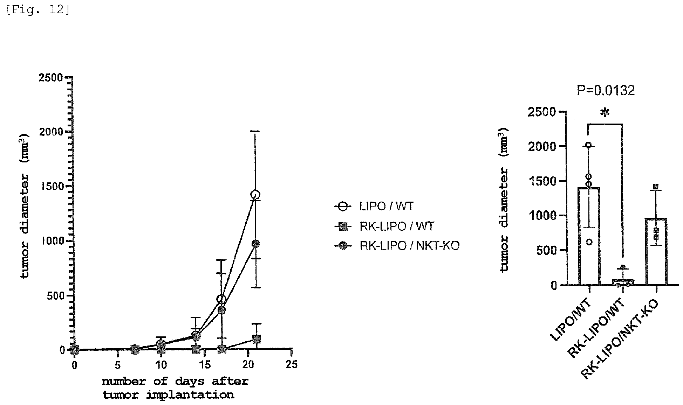

   Fig. 12 shows the results of Experimental Example 12. In the Figure, RK-LIPO/WT shows a group in which RK-163-containing liposome: (#7) was administered to C57BL/6 mice, RK-LIPO/NKT-KO shows a group in which RK-163-containing liposome: (#7) was administered to NKT-KO (NKT cell deficient) mouse (control), and LIPO/WT shows a group in which a liposome not containing RK-163 was administered to C57BL/6 mouse (control).

### [Description of Embodiments]

In the following, the present invention is described in detail.

### [1. Glycolipid (NKT cell ligand in the present invention)]

The liposome composition of the present invention contains a glycolipid selected from the group consisting of a compound represented by the formula (I), a compound represented by the formula (II), and salts thereof described below.

The compound represented by the formula (I), the compound represented by the formula (II), and salts thereof are NKT cell ligands. The NKT cell ligand refers to a compound that is specifically recognized by an NKT cell-specific antigen recognition receptor (NKT cell receptor) on the NKT cell when presented on a COld molecule, and can specifically activate the NKT cell. In the present specification, a compound represented by the formula (I), a compound represented by the formula (II), and a salt thereof are also collectively referred to as "the NKT cell ligand in the present invention".

First, a compound represented by the formula (I) (also to be referred to as compound (I) in the present specification) and a salt thereof are described: the formula (I): 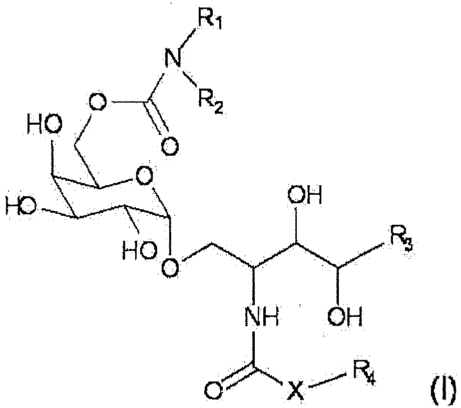
wherein X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30.

X is an alkylene group or -NH-. The "alkylene group" is, for example, a straight chain or branched alkylene group having a carbon number of 1 to 8. Specific examples include methylene, ethylene, trimethylene, tetramethylene, pentamethylene, hexamethylene, heptamethylene, octamethylene, propylene, ethylethylene, dimethylmethylene, dimethyltrimethylene and the like.

R₁ and R₂ are the same or different and each is a hydrogen atom, an alkyl group, a hydroxy group, an alkoxy group, or an aryl group optionally having substituent(s). R₁ and R₂ optionally form a 5- or 6-membered ring together with the adjacent nitrogen atom.

The "alkyl group" is, for example, a C₁₋₂₄, more preferably C₁₋₁₆, further preferably C₁₋₁₀, particularly preferably C₁₋₆ straight chain or branched alkyl group. Specific examples include methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl and the like. Preferred as the alkyl group for R₁ or R₂ is a C₁₋₆ alkyl group (e.g., methyl, ethyl).

The "alkoxy group" is, for example, a C₁₋₂₄, more preferably C₁₋₁₆, further preferably C₁₋₁₀, particularly preferably C₁₋₆, straight chain or branched alkoxy group. Specific examples include methoxy, ethoxy, propoxy, isopropoxy, butoxy, tert-butoxy and the like. Preferred as the alkoxy group for R₁ or R₂ is a C₁₋₆ alkoxy group (e.g., methoxy).

The "aryl group" in the "aryl group optionally having substituent (s)" is, for example, a C₆₋₁₄, more preferably C₆₋₁₂, monocyclic-tricyclic aryl group. Specific examples include phenyl, naphthyl, anthryl, phenanthryl and the like. Preferred as the aryl group for R₁ or R₂ is a C₆₋₁₂ aryl group (e.g., phenyl). Examples of the substituent that the "aryl group" optionally has include a halogen atom (e.g., chlorine atom, fluorine atom, bromine atom, iodine atom); an alkyl group (e.g., methyl, ethyl, n-propyl, isopropyl, n-butyl, isobutyl, sec-butyl, tert-butyl, pentyl, hexyl); a halogenoalkyl group (e.g., trifluoromethyl); an alkoxy group (e.g., methoxy, ethoxy, propoxy, isopropoxy, butoxy, tert-butoxy); a hydroxy group; an amino group; an alkylamino group (e.g., methylamino, dimethylamino, ethylamino, diethylamino); a cycloalkylamino group and the like. The position and number of the substituents are not particularly limited, and one to substitutable maximum number of substituents may be present at substitutable position(s).

The 5- or 6-membered ring optionally formed by R₁ and R₂ together with the adjacent nitrogen atom is, for example, a 5- or 6-membered nitrogen-containing saturated heterocycle, which is specifically pyrrolidine, piperidine, morpholine, thiomorpholine, piperazine or the like. Preferred is pyrrolidine, piperidine or morpholine.

R₃ is a hydrocarbon group having a carbon number of 1 to 20. The "hydrocarbon group having a carbon number of 1 to 20" is a concept encompassing a C₁₋₂₀ alkyl group, a C₂₋₂₀ alkenyl group, a C₂₋₂₀ alkynyl group, a C₃₋₂₀ cycloalkyl group, a C₃₋₂₀ cycloalkenyl group, and even a C₆₋₂₀ aryl group, which may be linear, branched or cyclic, or may be a saturated hydrocarbon group or an unsaturated hydrocarbon group, and optionally having an unsaturated bond in a molecule or at the terminal. Among these, preferred as R₃ are a C₁₋₂₀ alkyl group, a C₂₋₂₀ alkenyl group, and a C₂₋₂₀ alkynyl group, and more preferred is a C₁₂₋₁₄ alkyl group. As R₃, specifically, -(CH₂)₁₃CH₃ and the like can be mentioned.

R₄ is a hydrocarbon group having a carbon number of 1 to 30. The "hydrocarbon group having a carbon number of 1 to 30" is a concept encompassing a C₁₋₃₀ alkyl group, a C₂₋₃₀ alkenyl group, a C₂₋₃₀ alkynyl group, a C₃₋₃₀ cycloalkyl group, a C₃₋₃₀ cycloalkenyl group, and even a C₆₋₃₀ aryl group, which may be linear, branched or cyclic, or may be a saturated hydrocarbon group or an unsaturated hydrocarbon group, and optionally has an unsaturated bond in a molecule or at the terminal. Among these, preferred as R₄ are a C₁₋₃₀ alkyl group, a C₂₋₃₀ alkenyl group, and a C₂₋₃₀ alkynyl group, more preferred is a C₁₀₋₃₀ alkyl group, and further preferred is a C₁₅₋₂₅ alkyl group. Specific examples of R₄ include -(CH₂)₁₅CH₃, -(CH₂)₂₃CH₃, and the like.

The hydrocarbon group for R₃ or R₄ optionally has substituent(s). When the hydrocarbon group for R₃ or R₄ has substituent(s), examples of the substituent include an electron-donating group such as a halogen atom (preferably chlorine atom, fluorine atom); an alkoxy group (preferably C₁₋₂₄, more preferably C₁₋₁₆, still more preferably C₁₋₁₀, particularly preferably C₁₋₄) such as methoxy, ethoxy, propoxy, isopropoxy, butoxy, tert-butoxy and the like; an aryloxy group (preferably C₆₋₁₄) such as phenoxy and the like; a hydroxy group; an amino group; an alkylamino group such as methylamino, dimethylamino, ethylamino, diethylamino and the like; a cycloalkylamino group; an alkylcarbonylamino group such as acetamide and the like; a cycloalkylcarbonylamino group; arylcarbonylamino group (preferably, an arylcarbonylamino group wherein the aryl moiety is an aryl group having a carbon number of 6 to 14) such as benzoylamino and the like, and the like, further, an electron-withdrawing group such as a carboxy group; an alkoxycarbonyl group; an acyl group (acyl group is as mentioned below, preferably an alkyl-carbonyl group wherein the alkyl moiety is a straight chain or branched alkyl group having a carbon number of 1 to 24); a carbamoyl group; trifluoromethyl and the like. The position and number of the substituents are not particularly limited, and one to substitutable maximum number of substituents may be present at substitutable position(s). When one or more substituents are present, they may be the same or different.

The "acyl group" in the present specification is, for example, a formyl group; an alkyl-carbonyl group (e.g., an alkyl-carbonyl group wherein the alkyl moiety is a straight chain or branched alkyl group having a carbon number of 1 to 24 (preferably 1 to 12) (e.g., acetyl, propionyl, butyryl, isobutyryl, valeryl, pivaloyl, hexanoyl)); a cycloalkyl-carbonyl group (e.g., a cycloalkyl-carbonyl group wherein the cycloalkyl moiety is a cycloalkyl group having a carbon number of 3 to 10); an alkenyl-carbonyl group (e.g., an alkenyl-carbonyl group wherein the alkenyl moiety is a straight chain or branched alkenyl group having a carbon number of 2 to 12 (e.g., acryloyl, methacryloyl)); an aryl-carbonyl group (e.g., an aryl-carbonyl group wherein the aryl moiety is an aryl group having a carbon number of 6 to 14 (e.g., benzoyl, naphthoyl)) and the like. The aryl group of the aryl-carbonyl group is, for example, a monocyclic-tricyclic aromatic hydrocarbon group, and specific examples include phenyl, naphthyl, anthryl and phenanthryl. Among these, as the acyl group, formyl, acetyl, propionyl, butyryl, isobutyryl, benzoyl, naphthoyl and the like are preferred, and acetyl and benzoyl are more preferred.

Examples of the alkyl moiety of the above-mentioned alkylamino group and alkylcarbonylamino group include a straight chain or branched alkyl group (preferably having a carbon number of 1 to 24, more preferably a carbon number of 1 to 16, still more preferably a carbon number of 1 to 10, particularly preferably a carbon number of 1 to 4) such as methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl and the like.

Examples of the cycloalkyl moiety of the above-mentioned cycloalkylamino group and cycloalkylcarbonylamino group include a cycloalkyl group (preferably having a carbon number of 3 to 24, more preferably a carbon number of 3 to 16, still more preferably a carbon number of 3 to 10, particularly preferably a carbon number of 3 to 6) such as cyclopentyl, cyclohexyl and the like.

Examples of the alkoxy moiety of the above-mentioned alkoxycarbonyl group include those similar to the above-mentioned alkoxy group.

The above-mentioned substituents may be further substituted at substitutable position(s) by at least one kind from halogen, an alkyl group, a cycloalkyl group, an alkenyl group, an alkynyl group, a phenyl group, an alkoxy group, a hydroxy group, an amino group, an alkylamino group and a cycloalkylamino group.

Examples of the halogen, alkoxy group, alkylamino group and cycloalkylamino group include those similar to the above.

Examples of the alkyl group include an alkyl group (preferably having a carbon number of 1 to 24, more preferably a carbon number of 1 to 16, still more preferably a carbon number of 1 to 10, particularly preferably a carbon number of 1 to 4) such as methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl and the like.

Examples of the cycloalkyl group include a cycloalkyl group (preferably having a carbon number of 3 to 24, more preferably a carbon number of 3 to 16, still more preferably a carbon number of 3 to 10, particularly preferably a carbon number of 3 to 6) such as cyclopentyl, cyclohexyl and the like.

Examples of the alkenyl group include an alkenyl group (preferably having a carbon number of 2 to 24, more preferably a carbon number of 2 to 16, still more preferably a carbon number of 2 to 10, particularly preferably a carbon number of 2 to 4) such as vinyl, propenyl, butenyl and the like.

Examples of the alkynyl group include an alkynyl group (preferably having a carbon number of 2 to 24, more preferably a carbon number of 2 to 16, still more preferably a carbon number of 2 to 10, particularly preferably a carbon number of 2 to 4) such as ethynyl, propargyl, butynyl, pentynyl and the like.

In the present invention, the α configuration is employed from among the stereoisomers derived from the cyclic structure of sugar (galactopyranose).

When compound (I) has a stereoisomer derived from a structure other than a cyclic structure of sugar (e.g., asymmetric carbon etc. of a part other than the cyclic structure of sugar), any isomers are also encompassed in the present invention, which may be a mixture (including racemate) of two or more kinds of isomers at any ratio.

Particularly, compound (I) contains an optical isomer derived from the asymmetric carbon of a part other than the cyclic structure of sugar. In the present invention, compound (I) may be a single optically active form or a mixture of two or more kinds of optically active forms at any ratio (including racemates). The asymmetric carbon to which -NHC(=O)X-R⁴ is bonded is preferably in an S configuration, and the asymmetric carbon adjacent to the asymmetric carbon bonded to -NHC(=O)X-R₄, to which OH is bonded, is preferably in an R configuration. The asymmetric carbon to which R₃ is bonded is preferably in an R configuration.

Salts of compound (I) are preferably pharmaceutically acceptable salts; examples include inorganic acid salts such as hydrochlorides, hydrobromides, hydroiodides, sulfates, nitrates, and phosphates; organic acid salts such as succinates, fumarates, acetates, methanesulfonates, and toluenesulfonates; alkali metal salts such as sodium salts and potassium salts; alkaline earth metal salts such as magnesium salts and calcium salts; ammonium salts such as ammonium salts and alkylammonium salts; and the like.

Examples of preferred compound (I) or a salt thereof include the following.
(a) A compound of the formula (I), wherein
   X is -CH₂-;
   R₁ and R₂ are the same or different and each is a hydrogen atom, a C₁₋₆ alkyl group, a hydroxy group, or a C₁₋₆ alkoxy group;
   R₃ is a C₁₋₂₀ alkyl group; and
   R₄ is a C₁₋₃₀ alkyl group, or a salt thereof.
(b) A compound of the formula (I), wherein
   X is -CH₂-;
   R₁ and R₂ are the same or different and each is a hydrogen atom, a C₁₋₆ alkyl group, a hydroxy group, or a C₁₋₆ alkoxy group;
   R₃ is a C₁₂₋₁₄ alkyl group; and
   R₄ is a C₁₅₋₂₅ alkyl group, or a salt thereof.
(c) A compound of the formula (I), wherein
   X is -CH₂-;
   R₁ and R₂ are the same or different and each is a hydrogen atom, a C₁₋₆ alkyl group, or a hydroxy group;
   R₃ is a C₁₂₋₁₄ alkyl group; and
   R₄ is a C₁₅₋₂₅ alkyl group, or a salt thereof.
(d) A compound of the formula (I), wherein
   X is -CH₂-;
   R₁ and R₂ are the same or different and each is a hydrogen atom, methyl, or a hydroxy group;
   R₃ is -(CH₂)₁₃CH₃; and
   R₄ is -(CH₂)₂₃CH₃, or a salt thereof.

Specific examples of preferred compound (I) of the present invention are shown in Table 1 which is not to be construed as limitative.

**[Table 1]**

| | 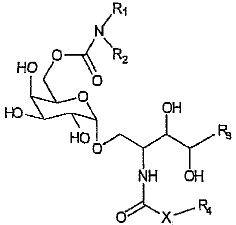 | | | |
|---|---|---|---|---|
| | -NR₁R₂ | -X- | -R₃ | -R₄ |
| RCAI-123 | -NMe₂ | -CH₂- | -C₁₄H₂₉ | -C₂₄H₄₉ |
| RCAI-124 | -NHMe | -CH₂- | -C₁₄H₂₉ | -C₂₄H₄₉ |
| RCAI-137 | -NHOH | -CH₂- | -C₁₄H₂₉ | -C₂₄H₄₉ |
| RCAI-138 | -NHOMe | -CH₂- | -C₁₄H₂₉ | -C₂₄H₄₉ |
| RCAI-148 | -NHEt | -CH₂- | -C₁₄H₂₉ | -C₂₄H₄₉ |
| RCAI-149 | -NEt₂ | -CH₂- | -C₁₄H₂₉ | -C₂₄H₄₉ |
| RCAI-121 | 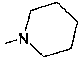 | -CH₂- | -C₁₄H₂₉ | -C₂₄H₄₉ |
| RCAI- 122 | 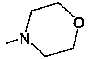 | -CH₂- | -C₁₄H₂₉ | -C₂₄H₄₉ |
| RCAI-131 | 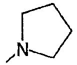 | -CH₂- | -C₁₄H₂₉ | -C₂₄H₄₉ |
| RCAI-132 | 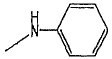 | -CH₂- | -C₁₄H₂₉ | -C₂₄H₄₉ |
| RCAI-139 | 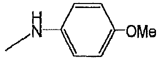 | -CH₂- | -C₁₄H₂₉ | -C₂₄H₄₉ |
| RCAI-140 | 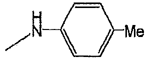 | -CH₂- | -C₁₄H₂₉ | -C₂₄H₄₉ |
| RCAI-141 | 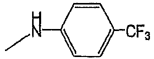 | -CH₂- | -C₁₄H₂₉ | -C₂₄H₄₉ |
| RCAI-150 | -NMe₂ | -NH- | -C₁₄H₂₉ | -C₁₆H₃₃ |

In the Table, -C₁₄H₂₉ is -(CH₂)₁₃CH₃, -C₂₄H₄₉ is -(CH₂)₂₃CH₃, and -C₁₆ H₃₃ is -(CH₂)₁₅CH₃.

Among them, the following compounds are preferred. RCAI-124 (also to be referred to as RK-163 in the present specification)

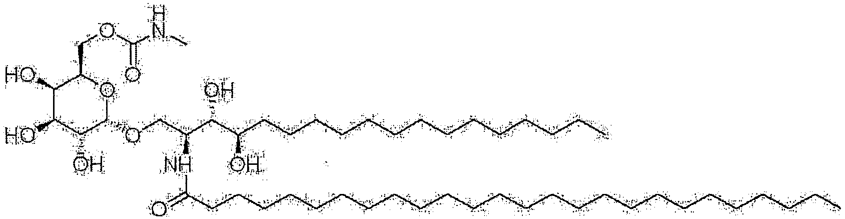

### RCAI-137

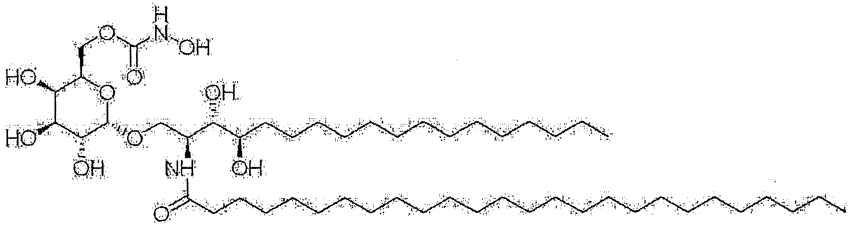

A compound represented by the formula (I) or a salt thereof can be produced by the methods disclosed in WO 2013/162016A1, US 2015/152128 A1, and the like.

A compound represented by the formula (II) (also to be referred to as compound (II) in the present specification) and a salt thereof are described next. the formula (II): 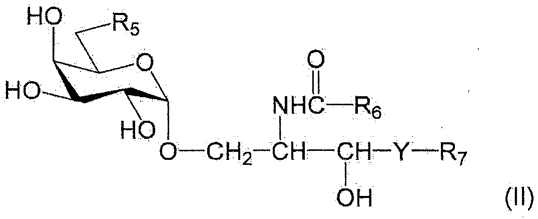 wherein R⁵ is a hydrogen atom, an alkyl group having a carbon number of 1 to 7, an alkoxy group having a carbon number of 1 to 6 or a halogen atom, R⁶ and R⁷ are each independently a substituted or unsubstituted hydrocarbon group having a carbon number of 1 to 28, and Y is -CH₂-, -CH(OH)- or -CH=CH-.

R₅ is a hydrogen atom, an alkyl group having a carbon number of 1 to 7, an alkoxy group having a carbon number of 1 to 6 or a halogen atom.

The alkyl group having a carbon number of 1 to 7 for R₅ is a substituted or unsubstituted alkyl group, and may form a ring. For example, methyl, ethyl, n-propyl, isopropyl, cyclopropyl, cyclopropylmethyl, n-butyl, isobutyl, sec-butyl, tert-butyl, pentyl, hexyl, heptyl, cyclohexylmethyl and the like can be mentioned, with preference given to methyl and ethyl.

The alkoxy group having a carbon number of 1 to 6 for R₅ is an oxygen atom bound with a substituted or unsubstituted alkyl group, wherein the alkyl moiety thereof may form a ring. For example, methoxy, ethoxy, n-propyloxy, isopropyloxy, cyclopropyloxy, cyclopropylmethyloxy, n-butoxy, isobutyloxy, sec-butyloxy, tert-butyloxy, pentyloxy, hexyloxy, cyclohexyloxy and the like can be mentioned, with preference given to methoxy, ethoxy and n-propyloxy.

Examples of the halogen atom for R₅ include a fluorine atom, a chlorine atom, a bromine atom and an iodine atom, with preference given to a fluorine atom and a chlorine atom.

R₆ and R₇ are each independently a substituted or unsubstituted hydrocarbon group having a carbon number of 1 to 28. In the present specification, the "hydrocarbon group" is a concept encompassing a substituted or unsubstituted alkyl group having a carbon number of 1 to 28, a substituted or unsubstituted alkenyl group having a carbon number of 2 to 28, a substituted or unsubstituted alkynyl group having a carbon number of 2 to 28, a substituted or unsubstituted cycloalkyl group having a carbon number of 3 to 28, a substituted or unsubstituted cycloalkenyl group having a carbon number of 3 to 28, a substituted or unsubstituted aryl group having a carbon number of 6 to 14, and a substituted or unsubstituted arylalkyl group having a carbon number of 7 to 28 (e.g., C₆₋₁₄ aryl-C₁₋₁₄ alkyl group), which may be in any of linear form, branched form and cyclic form, may be a saturated hydrocarbon group or an unsaturated hydrocarbon group, and may have an unsaturated bond either in a molecule or at the terminal. Among these, as R₆ and R₇, a substituted or unsubstituted alkyl group having a carbon number of 1 to 28 or a substituted or unsubstituted arylalkyl group having a carbon number of 7 to 28 are preferred.

When R₅ is a hydrogen atom, R₆ is preferably a substituted or unsubstituted hydrocarbon group having a carbon number of 24 to 28, and a substituted or unsubstituted alkyl group having a carbon number of 24 to 28 is further preferred.

Examples of the substituent of the hydrocarbon group for R₆ and R₇ include an electron-donating group such as a halogen atom (preferably chlorine atom, fluorine atom); an alkoxy group (preferably C₁₋₂₄, more preferably C₁₋₁₆, still more preferably C₁₋₁₀, particularly preferably C₁₋₄) such as a methoxy group, an ethoxy group, a propoxy group, an isopropoxy group, a butoxy group, a tert-butoxy group and the like; an aryloxy group (preferably C₆₋₁₄) such as a phenoxy group and the like; a hydroxy group; an amino group; an alkylamino group such as a methylamino group, a dimethylamino group, an ethylamino group, a diethylamino group and the like; a cycloalkylamino group; an alkylcarbonylamino group such as an acetamide group and the like; a cycloalkylcarbonylamino group; an arylcarbonylamino group (preferably, an arylcarbonylamino group wherein the aryl moiety is an aryl group having a carbon number of 6 to 14) such as benzoylamino group and the like, and the like, further, an electron-withdrawing group such as a carboxy group; an alkoxycarbonyl group; an acyl group (acyl group is as mentioned below, preferably an alkyl-carbonyl group wherein the alkyl moiety is a straight chain or branched alkyl group having a carbon number of 1 to 24); a carbamoyl group; a trifluoromethyl group and the like.

The "acyl group" in the present specification is, for example, a formyl group; an alkyl-carbonyl group (e.g., an alkyl-carbonyl group wherein the alkyl moiety is a straight chain or branched alkyl group having a carbon number of 1 to 24 (preferably 1 to 12) (e.g., acetyl group, propionyl group, butyryl group, isobutyryl group, valeryl group, pivaloyl group, hexanoyl group)); a cycloalkyl-carbonyl group (e.g., a cycloalkyl-carbonyl group wherein the cycloalkyl moiety is a cycloalkyl group having a carbon number of 3 to 10); an alkenyl-carbonyl group (e.g., an alkenyl-carbonyl group wherein the alkenyl moiety is a straight chain or branched alkenyl group having a carbon number of 2 to 12 (e.g., acryloyl group, methacryloyl group)); an aryl-carbonyl group (e.g., an aryl-carbonyl group wherein the aryl moiety is an aryl group having a carbon number of 6 to 14 (e.g., benzoyl group, naphthoyl group)) and the like. The aryl group of the aryl-carbonyl group is, for example, a monocyclic - tricyclic aromatic hydrocarbon group, and specific examples include a phenyl group, a naphthyl group, an anthryl group and a phenanthryl group. Among these, as the acyl group, a formyl group, an acetyl group, a propionyl group, a butyryl group, an isobutyryl group, a benzoyl group, a naphthoyl group and the like are preferred, and an acetyl group and a benzoyl group are more preferred.

Examples of the alkyl moiety of the above-mentioned alkylamino group and alkylcarbonylamino group include a straight chain or branched alkyl group (preferably having a carbon number of 1 to 24, more preferably a carbon number of 1 to 16, still more preferably a carbon number of 1 to 10, particularly preferably a carbon number of 1 to 4) such as a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group and the like.

Examples of the cycloalkyl moiety of the above-mentioned cycloalkylamino group and cycloalkylcarbonylamino group include a cycloalkyl group (preferably having a carbon number of 3 to 24, more preferably a carbon number of 3 to 16, still more preferably a carbon number of 3 to 10, particularly preferably a carbon number of 3 to 6) such as a cyclopentyl group, a cyclohexyl group and the like.

Examples of the alkoxy moiety of the above-mentioned alkoxycarbonyl group include those similar to the above-mentioned alkoxy group.

The above-mentioned substituents may be further substituted at substitutable position(s) by at least one kind from halogen, an alkyl group, a cycloalkyl group, an alkenyl group, an alkynyl group, a phenyl group, an alkoxy group, a hydroxy group, an amino group, an alkylamino group and a cycloalkylamino group.

Examples of the halogen, alkoxy group, alkylamino group and cycloalkylamino group include those similar to the above.

Examples of the alkyl group include an alkyl group (preferably having a carbon number of 1 to 24, more preferably a carbon number of 1 to 16, still more preferably a carbon number of 1 to 10, particularly preferably a carbon number of 1 to 4) such as a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group and the like.

Examples of the cycloalkyl group include a cycloalkyl group (preferably having a carbon number of 3 to 24, more preferably a carbon number of 3 to 16, still more preferably a carbon number of 3 to 10, particularly preferably a carbon number of 3 to 6) such as a cyclopentyl group, a cyclohexyl group and the like.

Examples of the alkenyl group include an alkenyl group (preferably having a carbon number of 2 to 24, more preferably a carbon number of 2 to 16, still more preferably a carbon number of 2 to 10, particularly preferably a carbon number of 2 to 4) such as a vinyl group, a propenyl group, a butenyl group and the like.

Examples of the alkynyl group include an alkynyl group (preferably having a carbon number of 2 to 24, more preferably a carbon number of 2 to 16, still more preferably a carbon number of 2 to 10, particularly preferably a carbon number of 2 to 4) such as an ethynyl group, a propargyl group, a butynyl group, a pentynyl group and the like.

Among these, as R₆, a substituted or unsubstituted alkyl group is preferred, and a linear alkyl group is preferred. As R₆, substituted or unsubstituted arylalkyl group is also preferred. When R₅ is an alkyl group having a carbon number of 1 to 7, an alkoxy group having a carbon number of 1 to 6 or a halogen atom, the carbon number of R₆ is preferably 18 to 26, more preferably 24 to 26. When R₅ is a hydrogen atom, the carbon number of R₆ is preferably 24 to 26. Specific examples of R₆ include -(CH₂)₂₃-CH₃, -(CH₂)₂₄-CH₃, -(CH₂)₂₅-CH₃, 10-(4-fluorophenyl)decyl, and the like.

In addition, as R₇, a substituted or unsubstituted alkyl group is preferred, and a linear alkyl group is preferred. The carbon number of R₇ is preferably 9 to 20, more preferably 12 to 18. Specific examples of R₇ include -(CH₂)₁₁-CH₃, -(CH₂)₁₂-CH₃, - (CH₂)₁₃-CH₃, - (CH₂)₁₄-CH₃, - (CH₂)₁₅-CH₃, - (CH₂)₁₆-CH₃, -(CH₂)₁₇-CH₃ and the like.

Y is -CH₂-, -CH(OH)- or -CH=CH-. Of these, -CH(OH)- is preferred.

When compound (II) has a stereoisomer, any isomers are also encompassed in the present invention, which may be a mixture (including racemate) of two or more kinds of isomers at any ratio.

Particularly, compound (II) contains an optical isomer derived from asymmetric carbon in the lipid moiety. In the present invention, they may be a single optically active form or a mixture (including racemate) of two or more kinds of optically active forms at any ratio. The asymmetric carbon to be bonded to -NHCOR₆ is preferably an S configuration. The asymmetric carbon having -OH and adjacent to the asymmetric carbon to be bonded to -NHCOR₆ is preferably an anti configuration relative to the asymmetric carbon to be bonded to -NHCOR₆. When Y is -CH(OH)-, the asymmetric carbon in -CH(OH)-for Y is preferably an R configuration.

Examples of the lipid moiety of compound (II) include 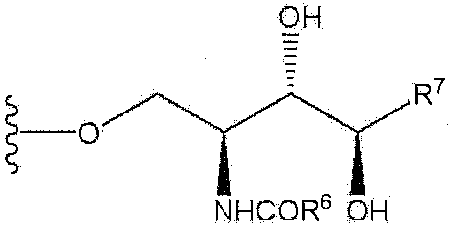 wherein each symbol is as defined above, and the like.

The salts of compound (II) are preferably pharmaceutically acceptable salts. Examples thereof include inorganic acid salts such as hydrochloride, hydrobromide, hydroiodide, sulfate, nitrate, phosphate and the like; organic acid salts such as succinate, fumarate, acetate, methanesulfonate, toluenesulfonate and the like; alkali metal salts such as sodium salt, potassium salt and the like; alkaline earth metal salts such as magnesium salt, calcium salt and the like; ammonium salts such as ammonium salt, alkylammonium salt and the like, and the like.

Specific preferable examples of compound (II) in the present invention are shown in, but are not limited to, Table 2.

**[Table 2]**

| compound No. | 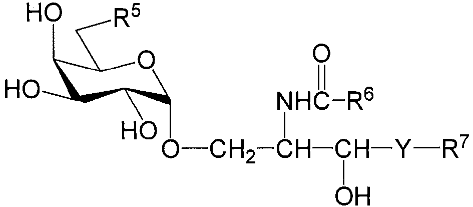 | | | |
|---|---|---|---|---|
| | R⁵ | R⁶ | Y | R⁷ |
| 2-9' | -OCH₃ | -(CH₂)₂₄CH₃ | 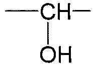 | -(CH₂)₁₃CH₃ |
| 3-4' | -CH₃ | -(CH₂)₂₄CH₃ | 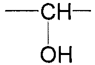 | -(CH₂)₁₃CH₃ |
| 3-7' | -F | -(CH₂)₂₄CH₃ | 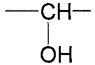 | -(CH₂)₁₃CH₃ |
| 3-10' | -H | -(CH₂)₂₄CH₃ | 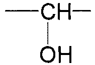 | -(CH₂)₁₃CH₃ |
| 3-13' | -OC₂H₅ | -(CH₂)₂₄CH₃ | 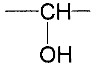 | -(CH₂)₁₃CH₃ |
| 3-16' | -O(CH)₂CH₃ | -(CH₂)₂₄CH₃ | 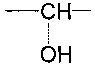 | -(CH₂)₁₃CH₃ |
| 165' | -OCH₃ | 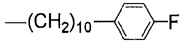 | 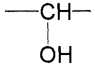 | -(CH₂)₁₃CH₃ |

Particularly, the following compounds are preferred. A compound represented by compound No. 2-9' in Table 2, wherein the steric structure is a compound represented by the following formula (RCAI-61):

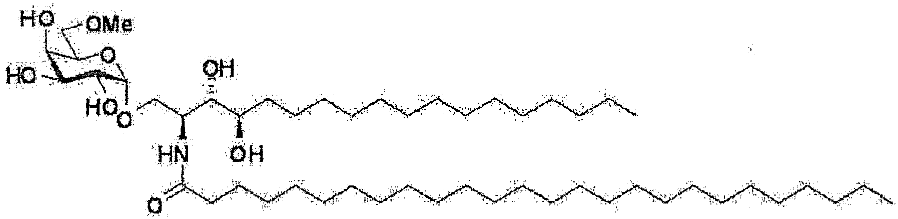

A compound represented by compound No. 3-4' in Table 2, wherein the steric structure is a compound represented by the following formula (RCAI-64):

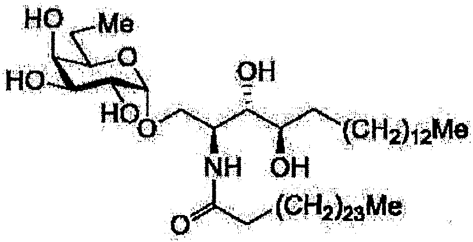

A compound represented by compound No. 3-13' in Table 2, wherein the steric structure is a compound represented by the following formula (RCAI-85):

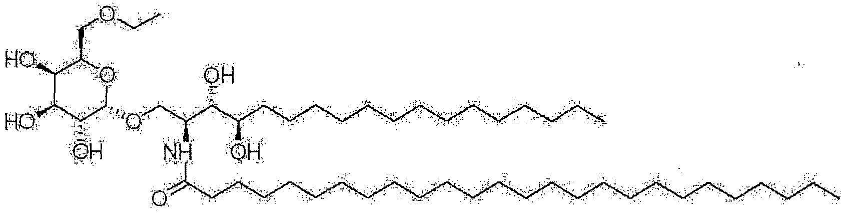

A compound represented by compound No. 165' in Table 2, wherein the steric structure is a compound represented by the following formula (RCAI-165):

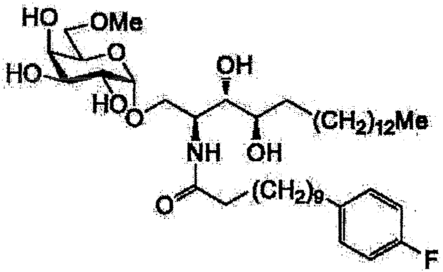

The compound represented by the formula (II) or a salt thereof can be produced by the methods described in WO 2009/119692 A1, US patent No. 8551959 and the like.

### [2. Liposome]

In the liposome composition of the present invention, the above-mentioned glycolipid is contained in liposomes.

In the present specification, a closed vesicle structure mainly composed of lipids (water-soluble particles obtained by aggregation of amphipathic molecules containing hydrophilic region and hydrophobic region) is called a liposome. Recently, it has also been called lipid nanoparticle (LNP), but in the present specification, the concept of liposome also includes those called lipid nanoparticles as long as they have the above-mentioned structure.

In the present invention, the liposome component may be any amphipathic molecule that can form a lipid closed vesicle structure by a known method, and lipids are preferred. Examples of the lipid in the present invention include phospholipids such as phosphatidyl choline (e.g., dioleoyl phosphatidyl choline, dilauroyl phosphatidyl choline, dimyristoyl phosphatidyl choline, dipalmitoyl phosphatidyl choline, distearoyl phosphatidyl choline), phosphatidyl glycerol (e.g., dioleoyl phosphatidyl glycerol, dilauroyl phosphatidyl glycerol, dimyristoyl phosphatidyl glycerol, dipalmitoyl phosphatidyl glycerol, distearoyl phosphatidyl glycerol), phosphatidyl ethanolamine (e.g., dioleoyl phosphatidyl ethanolamine, dilauroyl phosphatidyl ethanolamine, dimyristoyl phosphatidyl ethanolamine, dipalmitoyl phosphatidyl ethanolamine, distearoyl phosphatidyl ethanolamine), phosphatidyl serine, phosphatidyl inositol, phosphatidic acid, cardiolipin, and the like, sphingo glycolipid, glycerol glycolipid, cationic lipid (e.g., DOTAP, DLin-MC3-DMA ((6Z,9Z,28Z,31Z)-heptatriaconta-6,9,28,31-tetraen-19-yl-4-(dimethylamino)butanoate), ALC-0315 ([(4-hydroxybutyl)azanediyl]bis(hexane-6,1-diyl)bis(2-hexyldecanoic acid ester)), SM-102 (heptadecan-9-yl 8-((2-hydroxyethyl)(6-oxo-6-(undecyloxy)hexyl)amino)octanoic acid ester)) and the like. These may be used alone or two or more kinds thereof may be used or used in combination with non-polar substances such as cholesterol or lipid derivatives in which water-soluble polymers such as polyethylene glycol are bound to lipids, to prepare liposomes.

In the present invention, the liposome is preferably one that has affinity for antigen-presenting cells.

Examples of preferred liposomes in the present invention (examples of liposomes that have affinity for antigen-presenting cells) include the following liposomes (#2) to (#8).

### (#2) Liposome containing DOTAP, DOPE, and cholesterol

The weight ratio of DOTAP, DOPE, and cholesterol (DOTAP:DOPE:cholesterol) in the liposome (#2) is, for example, 1:0.1-10:0.1-10, preferably 1:0.3-3:0.2-2.

When liposome (#2) is used in the liposome composition of the present invention, the weight ratio of "NKT cell ligand in the present invention" and "total of DOTAP, DOPE, and cholesterol" is, for example, 1:1-10,000, preferably 1:1-1,000.

One embodiment of the liposome composition of the present invention using liposome (#2) is, for example, RK-163-containing liposome: (#2) described later.

### (#3) Liposome containing DOTAP, DOPE, MPEG2000-DMPE, Na salt, and cholesterol

The weight ratio of DOTAP, DOPE, MPEG2000-DMPE, Na salt, and cholesterol ((DOTAP:DOPE:MPEG2000-DMPE, Na salt:cholesterol) in the liposome (#3) is, for example, 1:0.1-10:0.1-10:0.1-10, preferably 1:0.3-3:0.2-2:0.2-2.

When liposome (#3) is used in the liposome composition of the present invention, the weight ratio of "NKT cell ligand in the present invention" and "total of DOTAP, DOPE, MPEG2000-DMPE, Na salt, and cholesterol" is, for example, 1:1-10,000, preferably 1:1-1,000.

One embodiment of the liposome composition of the present invention using liposome (#3) is, for example, RK-163-containing liposome: (#3) described later.

### (#4) Liposome containing DOTAP, DOPE, MPEG2000-DSPE, Na salt, and cholesterol

The weight ratio of DOTAP, DOPE, MPEG2000-DSPE, Na salt, and cholesterol (DOTAP:DOPE:MPEG2000-DSPE, Na salt:cholesterol) in the liposome (#4) is, for example, 1:0.1-10:0.1-10:0.1-10, preferably 1:0.3-3:0.2-2:0.2-2.

When liposome (#4) is used in the liposome composition of the present invention, the weight ratio of "NKT cell ligand in the present invention" and "total of DOTAP, DOPE, MPEG2000-DSPE, Na salt, and cholesterol" is, for example, 1:1-10,000, preferably 1:1-1,000.

One embodiment of the liposome composition of the present invention using liposome (#4) is, for example, RK-163-containing liposome: (#4) described later.

### (#5) Liposome containing HSPC, DSPG, and cholesterol

The weight ratio of HSPC, DSPG, and cholesterol (HSPC:DSPG:cholesterol) in the liposome (#5) is, for example, 1:0.1-10:0.1-10, preferably 1:0.2-2:0.2-2.

When liposome (#5) is used in the liposome composition of the present invention, the weight ratio of "NKT cell ligand in the present invention" and "total of HSPC, DSPG, and cholesterol" is, for example, 1:1-10,000, preferably 1:1-1,000.

One embodiment of the liposome composition of the present invention using liposome (#5) is, for example, RK-163-containing liposome: (#5) described later.

### (#6) Liposome containing HSPC, DOPS, and cholesterol

Liposome (#6) and liposome (#7) below have the same composition, but when used to produce NKT cell ligand-containing liposomes, the former is an example of an embodiment in which a relatively large amount is blended relative to the NKT cell ligand, and the latter is an example of an embodiment in which a relatively small amount is blended relative to the NKT cell ligand.

The weight ratio of HSPC, DOPS, and cholesterol (HSPC:DOPS:cholesterol) in the liposome (#6) is, for example, 1:0.1-10:0.1-10, preferably 1:0.2-2:0.2-2.

When liposome (#6) is used in the liposome composition of the present invention, the weight ratio of "NKT cell ligand in the present invention" and "total of HSPC, DOPS, and cholesterol" is, for example, 1:1-100,000, preferably 1:1-10,000.

One embodiment of the liposome composition of the present invention using liposome (#6) is, for example, RK-163-containing liposome: (#6) described later.

### (#7) Liposome containing HSPC, DOPS, and cholesterol

The weight ratio of HSPC, DOPS, and cholesterol (HSPC:DOPS:cholesterol) in the liposome (#7) is, for example, 1:0.1-10:0.1-10, preferably 1:0.2-2:0.2-2.

When liposome (#7) is used in the liposome composition of the present invention, the weight ratio of "NKT cell ligand in the present invention" and "total of HSPC, DOPS, and cholesterol" is, for example, 1:1-10,000, preferably 1:1-1,000.

One embodiment of the liposome composition of the present invention using liposome (#7) is, for example, RK-163-containing liposome: (#7), RCAI-61-containing liposome: (#9), or RCAI-137-containing liposome: (#10) described later.

### (#8) Liposome containing HSPC, MPEG2000-DSPE, Na salt, and cholesterol

The weight ratio of HSPC, MPEG2000-DSPE, Na salt, and cholesterol (HSPC:MPEG2000-DSPE, Na salt:cholesterol) in the liposome (#8) is, for example, 1:0.1-10:0.1-10, preferably 1:0.2-2:0.2-2.

When liposome (#8) is used in the liposome composition of the present invention, the weight ratio of "NKT cell ligand in the present invention" and "total of HSPC, MPEG2000-DSPE, Na salt, and cholesterol" is, for example, 1:1-10,000, preferably 1:1-1,000.

One embodiment of the liposome composition of the present invention using liposome (#8) is, for example, RK-163-containing liposome: (#8) described later.

In the present invention, among the above-mentioned liposomes, (#5) to (#8) are preferred, (#7) and (#8) are more preferred, and (#7) is further preferred.

### [3. Glycolipid (NKT cell ligand in the present invention)-containing liposome composition (the liposome composition of the present invention)]

The liposome constituting the liposome composition of the present invention can be prepared according to a known method. For example, the methods described in Liposome Technology, vol. 1, 2nd edition (by Gregory Gregoriadis (CRC Press, Boca Raton, Ann Arbor, London, Tokyo), Chapter 4, pp 67-80, Chapter 10, pp 167-184, and Chapter 17, pp 261-276 (1993))can be used. More specifically, examples include, but are not limited to, ultrasonication method, ethanol injecting method, French Press method, ether injection method, cholic acid method, calcium fusion method, freeze-thawing method, reversed-phase evaporation method, and the like.

Specifically, as shown in the Examples, the liposome components and the NKT cell ligand are each dissolved in an organic solvent in a glass test tube, nitrogen gas is sprayed onto the solution to evaporate the organic solvent, and the solution is completely dried in a desiccator (reduced pressure). An isotonic aqueous solvent (e.g., 9% sucrose aqueous solution, 5% glucose aqueous solution, phosphate buffered saline, etc.) is added to the dried solution, and the solution is emulsified with a homogenizer or the like to obtain an NKT cell ligand-containing liposome (liposome composition of the present invention). Furthermore, liposomes with a uniform particle size can be obtained by passing the liposome through a polycarbonate membrane filter (pore size 0.4 µm or 0.2 µm) under heating and pressurization. Finally, to ensure sterility, the liposome can also be passed through a general filter for sterilization (pore size 0.22 µm; for example, a PVDF membrane filter).

In the liposome composition of the present invention, the weight ratio of the "glycolipid selected from the group consisting of a compound represented by the formula (I), a compound represented by the formula (II), and salts thereof (NKT cell ligand in the present invention)" and "the total of other lipids (other than NKT cell ligand as the main drug) constituting liposome" is, for example, 1:1-100,000, preferably 1:1-10,000, more preferably 1:1-1,000, further preferably 1:1-100.

The average particle size of the liposome composition of the present invention is not particularly limited and is, for example, 10-1,000 nm, preferably 10-500 nm, more preferably 50-500 nm. The average particle size can be measured by the Quasi-Elastic Light scattering method.

The liposome composition of the present invention can be used alone or formulated by known methods together with pharmaceutically acceptable additives used generally (e.g., solvent, stabilizer, isotonicity agent, soothing agent, buffering agent, pH adjuster).

The liposome composition of the present invention is preferably formulated as an intravenous injection. When the liposome composition of the present invention is in the form of a liquid preparation such as injection and the like, it may be stored frozen or freeze-dried to remove water. A freeze-dried preparation is redissolved by adding distilled water for injection or the like before use and then used.

The liposome composition of the present invention can be used as an active ingredient of a medicament.

The liposome composition of the present invention is effective as an anti-malignant tumor agent.

Examples of the "malignant tumor" include liver cancer, esophageal cancer, gastric cancer, biliary tract cancer, pancreatic cancer, colorectal cancer, breast cancer, small cell lung cancer, non-small cell lung cancer, prostate cancer, bladder cancer, renal cancer, renal pelvic and ureteral cancer, penile cancer, retroperitoneal tumor, adrenal cancer, cancer of the head and neck, thyroid cancer, ovarian cancer, uterine cancer, skin cancer, malignant lymphoma, brain tumor, malignant melanoma, retinoblastoma, choroidal melanoma, intraocular lymphoma, eyelid tumor, lacrimal gland cancer, ocular adnexa lymphoma, orbital sarcoma, optic nerve tumor, pilocytic astrocytoma, diffuse astrocytoma, oligodendroglial tumor, glioblastoma, ependymoma, ganglioglioma, central neurocytoma, medulloblastoma, germinoma, primary central nervous system lymphoma, meningiomas, schwannoma, pituitary adenoma, craniopharyngioma, chordoma, hemangioblastoma, epidermoid tumor, neuroblastoma, oral cavity cancer, tongue cancer, adenoid cystic carcinoma, ear cancer, sarcoma of the head and neck sarcoma, male breast cancer, special types of breast cancer, malignant pericardial mesothelioma, malignant pleural mesothelioma, thymoma, mediastinal tumor, neuroendocrine tumor of the lung, malignant peritoneal mesothelioma, anal cancer, gastrointestinal neuroendocrine tumor, small intestine cancer, gastrointestinal membrane tumor, neuroendocrine tumor of the pancreas-gastrointestinal tract, pheochromocytoma, adrenocortical carcinoma, paraganglioma, malignant tunica vaginalis mesothelioma, tunica vaginalis tumor, vulvar cancer, uterine sarcoma, vaginal cancer, urachal cancer, peritoneal cancer, retroperitoneal sarcoma, malignant melanoma, basal cell cancer, extramammary Paget's disease, angiosarcoma of the skin, skin tumor, sweat gland cancer, sebaceous gland cancer, Merkel cell cancer, squamous cell cancer, uterine sarcoma, osteosarcoma, rhabdomyoma, Ewing's sarcoma, endocardial sarcoma, angiosarcoma, truncal sarcoma, desmoid tumor, sarcoma of the head and neck, soft tissue sarcoma, bone cyst, cheek bone cyst, enchondroma, osteochondroma, osteoclastoma, osteosarcoma, chondrosarcoma, chordoma, myxofibrosarcoma, liposarcoma, leiomyosarcoma, rhabdomyosarcoma, malignant peripheral nerve sheath tumor, epithelioid sarcoma, clear cell sarcoma, synovial sarcoma, malignant fibrous histiocytoma, alveolar soft part sarcoma, dermatofibrosarcoma protuberans, adult T cells leukemia lymphoma, extranodal T-/NK-cell lymphoma, nasal type, Burkitt's lymphoma, skin lymphoma, Hodgkin lymphoma, peripheral T-cell lymphoma, chronic lymphocytic leukemia, neuroblastoma, kidney tumor, germ cell tumor, fibrosarcoma, medullary cancer, clear cell sarcoma, ileal cancer, cancer of the external auditory canal, thymic cancer, cancer of the hair follicle, oligoastrocytoma, uveal malignant melanoma, myxofibrosarcoma, soft tissue sarcoma, enchondroma, endometrial stromal sarcoma, cancer of the internal auditory canal, aneurysmal bone cyst, cancer of the middle ear canal, glioma, neurofibroma, lipoma, and duodenal cancer.

The liposome composition of the present invention is effective as an anti-infective agent.

Examples of the "infectious disease" include viral disease (e.g., viral hepatitis due to hepatitis B virus, hepatitis C virus, or hepatitis D virus, herpes, epidemic influenza, novel coronavirus infection, Ebola hemorrhagic fever, yellow fever, rabies, Zika virus, SARS, MARS, dengue fever, Japanese encephalitis, polio, Lassa fever, varicella, Streptococcus pneumoniae, measles), bacterium infectious disease (e.g., drug-resistant tuberculosis, atypical mycobacterium infection, cholera, diphtheria, typhus), and fungus disease (e.g., candidiasis, toxoplasmosis, trypanosomiasis).

The liposome composition of the present invention is effective as an NKT cell activator, an inducer for IFN-γ production from NKT cell, an activator for innate immune system lymphocytes and acquired immune system lymphocytes, or a long-term immune memory inducer.

The liposome composition of the present invention can be safely administered to human, mammals other than human (e.g., mouse, rat, rabbit, dog, cat, bovine, horse, monkey, swine, etc.).

The subject of administration of the liposome composition of the present invention is suitably a human or a mammal other than human affected with malignant tumor, or a human or a mammal other than human affected with an infectious disease or at a risk of having an initial infectious disease. The liposome preparation of the present invention is also applicable to the treatment or prophylaxis of infectious disease or metastasis or recurrence of malignant tumor.

NKT cells can activate various immune cells (e.g., cytotoxic T cells, NK cells, etc.) that act directly on malignant tumor cells and pathogen-infected cells in the body. Thus, the liposome composition of the present invention may be applicable to the treatment of malignant tumor and infectious diseases irrespective of the kind of malignant tumor or pathogens.

The administration method of the liposome composition of the present invention is not limited as long as it can make the liposome composition of the present invention reach antigen-presenting cells existing in or around the affected part. For example, mainly intravenous administration, and further, intraarterial administration, intramucosal administration, administration into lymph node, administration into affected part tissue and the like can be mentioned. In general, the liposome composition is administered intravenously to allow delivery thereof to the lungs and liver, where NKT cells are abundant, thereby artificially activating NKT cells. In some cases, the liposome composition can be delivered directly to the disease site of the patient through an injection needle, or the liposome composition can also be administered into vein through a catheter.

The dosage of the liposome composition of the present invention can be appropriately set according to the administration route, severity of symptoms, age of the patient, degree of side effects, and the like.

The dosage of the liposome composition of the present invention in the amount of "a glycolipid selected from the group consisting of a compound represented by the formula (I), a compound represented by the formula (II), and salts thereof" is, for example, about 12 to 400 ng, preferably about 40 to 400 ng, per kg body weight for the first administration, in which one course is completed by re-administering the same amount four weeks after the first administration. Thereafter, the number of CD8 immune memory T cells is followed up by progress observation, and "two administrations at four-week intervals" are continuously repeated with the aim of maintaining the number of memory cells at or above the level before treatment.

The liposome composition of the present invention can be efficiently delivered to antigen-presenting cells (e.g., dendritic cells) in a living body by intravenously administering to the living body. Therefore, the present invention also relates to "a method for delivering the glycolipid to an antigen-presenting cell, comprising administering a liposome composition comprising a glycolipid selected from the group consisting of a compound represented by the formula (I), a compound represented by the formula (II), and salts thereof to the target".

The present invention is explained in more detail in the following based on Examples and Experimental Examples; however, the present invention is not limited to them.

### [Example]

RK-163, RCAI-61, and RCAI-137 used in Example are compounds represented by the aforementioned chemical structural formulas.

Abbreviations used in the present specification mean the following.
HSPC: Hydrogenated soybean phosphatidycholine
DOTAP: 1,2-dioleoyloxy-3-trimethylammonium propane chloride
DOPE: 1,2-dioleoyl-sn-glycero-3-phosphoethanolamine MPEG2000-DMPE, Na salt: N-(carbonyl-methoxypolyethyleneglycol 2000)-1,2-dimyristoyl-sn-glycero-3-phosphoethanolamine, sodium salt
MPEG2000-DSPE, Na salt: N-(carbonyl-methoxypolyethyleneglycol 2000)-1,2-distearoyl-sn-glycero-3-phosphoethanolamine, sodium salt
DSPG: 1,2-distearoyl-sn-glycero-3-phospho-(1'-rac-glycerol)
DOPS: 1,2-dioleoyl-sn-glycero-3-phospho-L-serine
PEG: polyethyleneglycol
PS: phosphathydylserine
OVA: Ovalbumin
PBS: phosphate buffered saline

### [Preparation of sample #1 (= RK-163-containing CAPTISOL aqueous solution: (#1))]

Using the reagents and ingredients shown in Table 3 and according to the following preparation method, sample #1 for safety test and main pharmacological test was prepared.

### (Composition of prepared sample)

### · For safety test

15 ng/mL RK-163, 177 ng/mL CAPTISOL in 9% sucrose aqueous solution

### · For main pharmacological test

1.5 ng/mL RK-163, 17.7 ng/mL CAPTISOL in 9% sucrose aqueous solution

**[Table 3]**

| | Manufacturing company or preparation method | Cat No. | Lot No. |
|---|---|---|---|
| CAPTISOL (trade name) | CyDex Pharmaceuticals. Inc. | - | NC-04A-170167 |
| chloroform | Wako Pure Chemical Industries, Ltd. (*) | 038-02606 | ESL3428 |
| methanol | Wako Pure Chemical Industries, Ltd. | 130-16585 | APF2160 |
| RK-163 | provided by RIKEN | | F-01 |
| 9% sucrose aqueous solution | prepared by dissolving sucrose at 9% in ultrapure water, and passing through filter for filtration sterilization with pore size 0.22 µm | | |

| | | | |
|---|---|---|---|
| * Wako Pure Chemical Industries, Ltd.: now FUJIFILM Wako Pure Chemical Corporation, hereinafter the same | | | |

### (Preparation method)

### · For safety test

RK-163 was dissolved in a solution of methanol:chloroform = 1:2 (v/v) at 100 ng/mL. 1.5 mL of the solution was placed in a glass bottle, dried by blowing nitrogen gas while heating, and then left standing in a desiccator. CAPTISOL was dissolved in a sucrose aqueous solution at 177 ng/mL, and 10 mL thereof was added to the glass bottle containing the dried RK-163 to solubilize the RK-163. The solution was passed through a filter for filtration sterilization with 0.22 µm pore size and stored at 4°C in a glass bottle with a screw cap. This solution was used as sample #1 (for safety test).
- For main pharmacological test
   1 mL of sample #1 (for safety test) was taken in another glass bottle, and 9 mL of 9% sucrose aqueous solution was added and diluted by mixing. The solution was passed through a PVDF membrane filter for filtration sterilization with 0.22 µm pore size and stored at 4°C in a glass bottle with a screw cap.

This solution was used as sample #1 (for main pharmacological test).

### [Preparation of sample #2 (= RK-163-containing liposome: (#2))]

Using the reagents and ingredients shown in Table 4 and according to the following preparation method, sample #2 for safety test and main pharmacological test was prepared.

### (Composition of prepared sample)

### · For safety test

15 ng/mL RK-163, 203 ng/mL total lipid (all lipids other than RK-163; hereinafter the same) in 9% sucrose aqueous solution lipid composition: DOTAP 91.7 ng/mL, DOPE 73.2 ng/mL, cholesterol 38.1 ng/mL

### · For main pharmacological test

1.5 ng/mL RK-163, 20.3 ng/mL total lipid in 9% sucrose aqueous solution
lipid composition: DOTAP 9.17 ng/mL, DOPE 7.32 ng/mL, cholesterol 3.81 ng/mL
particle size: 141.4 nm PDI: 0.1218

**[Table 4]**

| | Manufacturing company or preparation method | Cat No. | Lot No. |
|---|---|---|---|
| chloroform | Wako Pure Chemical Industries, Ltd. | 038-02606 | ESL3428 |
| methanol | Wako Pure Chemical Industries, Ltd. | 130-16585 | APF2160 |
| RK-163 | provided by RIKEN | | F-01 |
| DOTAP | Merck & Co. | CH2900029 | MBR0005-01 |
| DOPE | NIPPON FINE CHEMICAL CO., LTD. | - | ROK-MD061 |
| Cholesterol | KANTO CHEMICAL CO., INC. | 07331-30 | 901B2144 |
| 9% sucrose aqueous solution | prepared by dissolving sucrose at 9% in ultrapure water, and passing through filter for filtration sterilization with pore size 0.22 µm | | |

### (Preparation method)

### · For safety test

DOTAP, DOPE, and cholesterol were placed in one flask at 91.7 mg, 73.2 mg, and 38.1 mg, respectively, and dissolved in 100 mL of a solution of methanol:chloroform = 1:2 (v/v). 1.0 mL of the solution was mixed with 1.5 mL of a solution of RK-163 (100 µg/mL in methanol:chloroform = 1:2), and the mixture was dried by blowing nitrogen while heating, and then left standing in a desiccator. 10 mL of a 9% sucrose aqueous solution was added to the dried mixture, and the mixture was emulsified with a Teflon homogenizer and further sized with an extruder (heated to 50°C, polycarbonate membrane filter with a pore size of 0.2 µm, one sheet). The prepared liposome dispersion was further diluted 1000-fold with a 9% sucrose aqueous solution, filtered with a PVDF membrane filter for filtration sterilization with a sucrose pore size of 0.22 µm, placed in a glass bottle with a screw cap, and stored at 4°C. This solution was used as sample #2 (for safety test).

### · For main pharmacological test

1 mL of sample #2 (for safety test) was taken in another glass bottle, and 9 mL of 9% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #2 (for main pharmacological test).

### [Preparation of sample #3 (= RK-163-containing liposome: (#3))]

Using the reagents and ingredients shown in Table 5 and according to the following preparation method, sample #3 for safety test and main pharmacological test was prepared.

### (Composition of prepared sample)

### · For safety test

15 ng/mL RK-163, 247.2 ng/mL total lipid in 9% sucrose aqueous solution
lipid composition: DOTAP 91.7 ng/mL, DOPE 73.2 ng/mL, MPEG2000-DMPE, Na salt 44.2 ng/mL, cholesterol 38.1 ng/mL

### · For main pharmacological test

1.5 ng/mL RK-163, 24.7 ng/mL total lipid in 9% sucrose aqueous solution
lipid composition: DOTAP 9.17 ng/mL, DOPE 7.32 ng/mL, MPEG2000-DMPE, Na salt 4.42 ng/mL, cholesterol 3.81 ng/mL particle size: 126.7 nm PDI: 0.05407

**[Table 5]**

| | Manufacturing company or preparation method | Cat No. | Lot No. |
|---|---|---|---|
| chloroform | Wako Pure Chemical Industries, Ltd. | 038-02606 | ESL3428 |
| methanol | Wako Pure Chemical Industries, Ltd. | 130-16585 | APF2160 |
| RK-163 | provided by RIKEN | | F-01 |
| DOTAP | Merck & Co. | CH2900029 | MBR0005-01 |
| DOPE | NIPPON FINE CHEMICAL CO., LTD. | - | ROK-MD061 |
| Cholesterol | KANTO CHEMICAL CO., INC. | 07331-30 | 901B2144 |
| MPEG2000-DMPE, Na salt | NOF CORPORATION | PM-020CN | M195506 |
| 9% sucrose aqueous solution | prepared by dissolving sucrose at 9% in ultrapure water, and passing through filter for filtration sterilization with pore size 0.22 µm | | |

### (Preparation method)

### · For safety test

DOTAP, DOPE, MPEG2000-DMPE, Na salt, and cholesterol were weighed in one flask at 9.17 mg, 7.32 mg, 4.42 mg, and 3.81 mg, respectively, and dissolved in 10 mL of a solution of methanol:chloroform = 1:2 (v/v). 1.0 mL of the solution was mixed with 1.5 mL of a solution of RK-163 (100 µg/mL in methanol:chloroform = 1:2), and the mixture was dried by blowing nitrogen while heating, and then left standing in a desiccator. 10 mL of a 9% sucrose aqueous solution was added to the dried mixture, and the mixture was emulsified with a Teflon homogenizer and further sized with an extruder (heated to 50°C, polycarbonate membrane filter with a pore size of 0.2 µm, one sheet). The prepared liposome dispersion was further diluted 1000-fold with a 9% sucrose aqueous solution, filtered with a PVDF membrane filter for filtration sterilization with a pore size of 0.22 µm, placed in a glass bottle with a screw cap, and stored at 4°C. This solution was used as sample #3 (for safety test).

### · For main pharmacological test

1 mL of sample #3 (for safety test) was taken in another glass bottle, and 9 mL of 9% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #3 (for main pharmacological test).

### [Preparation of sample #4 (= RK-163-containing liposome: (#4))]

Using the reagents and ingredients shown in Table 6 and according to the following preparation method, sample #4 for safety test and main pharmacological test was prepared.

### (Composition of prepared sample)

### · For safety test

15 ng/mL RK-163, 249 ng/mL total lipid in 9% sucrose aqueous solution
lipid composition: DOTAP 91.7 ng/mL, DOPE 73.2 ng/mL, MPEG2000-DSPE, Na salt 46 ng/mL, cholesterol 38.1 ng/mL

### · For main pharmacological test

1.5 ng/mL RK-163, 24.9 ng/mL total lipid in 9% sucrose aqueous solution
lipid composition: DOTAP 9.17 ng/mL, DOPE 7.32 ng/mL, MPEG2000-DSPE, Na salt 4.6 ng/mL, cholesterol 3.81 ng/mL
particle size: 126.2 nm PDI: 0.09744

**[Table 6]**

| | Manufacturing company or preparation method | Cat No. | Lot No. |
|---|---|---|---|
| chloroform | Wako Pure Chemical Industries, Ltd. | 038-02606 | ESL3428 |
| methanol | Wako Pure Chemical Industries, Ltd. | 130-16585 | APF2160 |
| RK-163 | provided by RIKEN | | F-01 |
| DOTAP | Merck & Co. | CH2900029 | MBR0005-01 |
| DOPE | NIPPON FINE CHEMICAL CO., LTD. | - | ROK-MD061 |
| Cholesterol | KANTO CHEMICAL CO., INC. | 07331-30 | 901B2144 |
| MPEG2000-DSPE, Na salt | Lipoid | LIPOID PE 18:0/18:0-PEG 2000 Batch No. 588200-2190092-01/030 | |
| 9% sucrose aqueous solution | prepared by dissolving sucrose at 9% in ultrapure water, and passing through filter for filtration sterilization with pore size 0.22 µm | | |

### (Preparation method)

### · For safety test

DOTAP, DOPE, MPEG2000-DSPE, Na salt, and cholesterol were placed in one flask at 9.17 mg, 7.32 mg, 4.6 mg, and 3.81 mg, respectively, and dissolved in 10 mL of a solution of methanol:chloroform = 1:2 (v/v). 1.0 mL of the solution was mixed with 1.5 mL of a solution of RK-163 (100 µg/mL in methanol:chloroform = 1:2), and the mixture was dried by blowing nitrogen while heating, and then left standing in a desiccator. 10 mL of a 9% sucrose aqueous solution was added to the dried mixture, and the mixture was emulsified with a Teflon homogenizer and further sized with an extruder (heated to 50°C, polycarbonate membrane filter with a pore size of 0.2 µm, one sheet). The prepared liposome dispersion was further diluted 1000-fold with a 9% sucrose aqueous solution, filtered with a PVDF membrane filter for filtration sterilization with a pore size of 0.22 µm, placed in a glass bottle with a screw cap, and stored at 4°C. This solution was used as sample #4 (for safety test).

### • For main pharmacological test

1 mL of sample #4 (for safety test) was taken in another glass bottle, and 9 mL of 9% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #4 (for main pharmacological test).

### [Preparation of sample #5 (= RK-163-containing liposome: (#5))]

Using the reagents and ingredients shown in Table 7 and according to the following preparation method, sample #5 for safety test and main pharmacological test was prepared.

### (Composition of prepared sample)

### · For safety test

322 ng/mL RK-163, 3222 ng/mL total lipid in 10% sucrose aqueous solution
(Each concentration was calculated by quantifying cholesterol in the prepared sample and converting same to a total lipid concentration based on the weight ratio at the time of charging.)

### • For main pharmacological test

32.2 ng/mL RK-163, 322.2 ng/mL total lipid in 10% sucrose aqueous solution

### · Lipid composition (the above-mentioned lipid concentration of safety test sample):

HSPC 1953 ng/mL
DSPG 792 ng/mL
cholesterol 475 ng/mL
(Each concentration was calculated by quantifying cholesterol in the prepared sample and converting same to each lipid concentration based on the weight ratio at the time of charging.)
average particle size: 191.4 nm PDI: 0.083

**[Table 7]**

| | Manufacturing company or preparation method | Cat No. | Lot No. |
|---|---|---|---|
| chloroform | SAJ | 05-3400-5 | G5835 |
| methanol | SAJ | 19-2410-5 | J4847 |
| RK-163 | provided by RIKEN | | F-01 |
| HSPC | NOF CORPORATION | | 150107 |
| DSPG | NOF CORPORATION | | 1112851L |
| Cholesterol | NOF CORPORATION | | 1009CH02 |
| 10% sucrose aqueous solution | prepared by dissolving sucrose at 10% in ultrapure water, and passing through filter for filtration sterilization with pore size 0.22 µm | | |

### (Preparation method)

### · For safety test

HSPC, DSPG, and cholesterol were placed in one flask at 6.66 mg, 2.7 mg, and 1.61 mg, respectively, and dissolved in 1.5 mL of a solution of methanol:chloroform = 1:1 (v/v). 1.5 mL of the solution was mixed with 0.22 mL of a solution of RK-163 (5 mg/mL in methanol:chloroform = 1:1), and the mixture was dried by blowing nitrogen while heating, and then left standing in a desiccator. 2.42 mL of a 10% sucrose aqueous solution was added to the dried mixture, and the mixture was sized with an Extruder (heated to 50°C, pore size 400 nm, once, pore size 200 nm, twice) and filtered with a 0.22 µm PVDF membrane filter, and the particle size was adjusted. The prepared liposome dispersion was placed in a glass bottle with a screw cap.

This liposome dispersion was further diluted 1000-fold with a 10% sucrose aqueous solution, filtered with a PVDF membrane filter for filtration sterilization with a pore size of 0.22 µm, placed in a glass bottle with a screw cap, and stored at 4°C. This solution was used as sample #5 (for safety test).

### · For main pharmacological test

### 1 mL of sample #5 (for safety test) was taken in another glass bottle, and 9 mL of 10% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #5 (for main pharmacological test). Since the RK-163 concentration of the main pharmacological test sample was 32.2 ng/mL, the sample was further diluted with PBS and the RK-163 concentration was set to 1.5 ng/mL in the experiment.

### [Preparation of sample #6 (= RK-163-containing liposome: (#6))]

Using the reagents and ingredients shown in Table 8 and according to the following preparation method, sample #6 for safety test and main pharmacological test was prepared.

### (Composition of prepared sample)

### · For safety test

15 ng/mL RK-163, 221 µg/mL total lipid in 9% sucrose aqueous solution
lipid composition: HSPC 103 µg/mL DOPS 79.7 µg/mL cholesterol 38.1 µg/mL

### · For main pharmacological test

1.5 ng/mL RK-163, 22.1 µg/mL lipid in 9% sucrose aqueous solution
lipid composition: HSPC 10.3 µg/mL DOPS 7.97 µg/mL cholesterol 3.81 µg/mL
particle size: 168.8 nm PDI: 0.1288

**[Table 8]**

| | Manufacturing company or preparation method | Cat No. | Lot No. |
|---|---|---|---|
| chloroform | Wako Pure Chemical Industries, Ltd. | 038-02606 | ESL3428 |
| methanol | Wako Pure Chemical Industries, Ltd. | 130-16585 | APF2160 |
| RK-163 | provided by RIKEN | | F-01 |
| LIPOID S PC 3[Hydrogenated soybean phosphatidylcholine: HSPC] | Lipoid | LIPOID S PC-3 Batch No. 525600-2190668-01/023 | |
| DOPS | NIPPON FINE CHEMICAL CO., LTD. | | RGF-NF 231 |
| Cholesterol | KANTO CHEMICAL CO., INC. | 07331-30 | 901B2144 |
| 9% sucrose aqueous solution | prepared by dissolving sucrose at 9% in ultrapure water, and passing through filter for filtration sterilization with pore size 0.22 µm | | |

### (Preparation method)

### · Safety test

HSPC, DOPS, and cholesterol were placed in one flask at 10.3 mg, 7.97 mg and 3.81 mg, respectively, and dissolved in 10 mL of a solution of methanol:chloroform = 1:2 (v/v). 1.0 mL of the solution was mixed with 1.5 mL of a solution of RK-163 (100 ng/mL in methanol:chloroform = 1:2), and the mixture was dried by blowing nitrogen while heating, and then left standing in a desiccator. 10 mL of a 9% sucrose aqueous solution was added to the dried mixture, and the mixture was emulsified with a Teflon homogenizer and further sized with an extruder (heated to 50°C, polycarbonate membrane filter with a pore size of 0.2 µm, one sheet). The prepared liposome dispersion was filtered with a PVDF membrane filter for filtration sterilization with a pore size of 0.22 µm, placed in a glass bottle with a screw cap, and stored at 4°C. This solution was used as sample #6 (for safety test).

### · For main pharmacological test

1 mL of sample #6 (for safety test) was taken in another glass bottle, and 9 mL of 9% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #6 (for main pharmacological test).

### [Preparation of sample #7 (= RK-163-containing liposome: (#7))]

Using the reagents and ingredients shown in Table 9 and according to the following preparation method, sample #7 for safety test and main pharmacological test was prepared.

### (Composition of prepared sample)

### · For safety test

15 ng/mL RK-163, 220.8 ng/mL total lipid in 9% sucrose aqueous solution
lipid composition: HSPC 103 ng/mL, DOPS 79.7 ng/mL, cholesterol 38.1 ng/mL

### · For main pharmacological test

1.5 ng/mL RK-163, 22.08 ng/mL total lipid in 9% sucrose aqueous solution
lipid composition: HSPC 10.3 ng/mL, DOPS 7.97 ng/mL, cholesterol 3.81 ng/mL
particle size: 145.7 nm PDI: 0.06813

**[Table 9]**

| | Manufacturing company or preparation method | Cat No. | Lot No. |
|---|---|---|---|
| chloroform | Wako Pure Chemical Industries, Ltd. | 038-02606 | ESL3428 |
| methanol | Wako Pure Chemical Industries, Ltd. | 130-16585 | APF2160 |
| RK-163 | provided by RIKEN | | F-01 |
| HSPC | Lipoid | LIPOID S PC-3 Batch No. 525600-2190668-01/023 | |
| DOPS | NIPPON FINE CHEMICAL CO., LTD. | - | RGF-NF 231 |
| Cholesterol | KANTO CHEMICAL CO., INC. | 07331-30 | 901B2144 |
| 9% sucrose aqueous solution | prepared by dissolving sucrose at 9% in ultrapure water, and passing through filter for filtration sterilization with pore size 0.22 µm | | |

### (Preparation method)

### · Safety test

HSPC, DOPS, and cholesterol were placed in one flask at 10.3 mg, 7.97 mg, and 3.81 mg, respectively, and dissolved in 10 mL of a solution of methanol:chloroform = 1:2 (v/v). 1.0 mL of the solution was mixed with 1.5 mL of a solution of RK-163 (100 µg/mL in methanol:chloroform = 1:2), and the mixture was dried by blowing nitrogen while heating, and then left standing in a desiccator. 10 mL of a 9% sucrose aqueous solution was added to the dried mixture, and the mixture was emulsified with a Teflon homogenizer and further sized with an extruder (heated to 50°C, polycarbonate membrane filter with a pore size of 0.2 µm, one sheet). The prepared liposome dispersion was further diluted 1000-fold with a 9% sucrose aqueous solution, filtered with a PVDF membrane filter for filtration sterilization with a pore size of 0.22 µm, placed in a glass bottle with a screw cap, and stored at 4°C. This solution was used as sample #7 (for safety test).

### · For main pharmacological test

1 mL of sample #7 (for safety test) was taken in another glass bottle, and 9 mL of 9% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #7 (for main pharmacological test).

### [Preparation of sample #8 (= RK-163-containing liposome: (#8))]

Using the reagents and ingredients shown in Table 10 and according to the following preparation method, sample #8 for safety test and main pharmacological test was prepared.

### (Composition of prepared sample)

### · For safety test

15 ng/mL RK-163, 242.6 ng/mL total lipid in 9% sucrose aqueous solution
lipid composition: HSPC 145 ng/mL, MPEG2000-DSPE, Na salt 48.8 ng/mL, cholesterol 48.8 ng/mL

### · For main pharmacological test

1.5 ng/mL RK-163, 24.26 ng/mL total lipid in 9% sucrose aqueous solution
lipid composition: HSPC 14.5 ng/mL, MPEG2000-DSPE, Na salt 4.88 ng/mL, cholesterol 4.88 ng/mL
particle size: 143.0 nm PDI: 0.05755

**[Table 10]**

| | Manufacturing company or preparation method | Cat No. | Lot No. |
|---|---|---|---|
| chloroform | Wako Pure Chemical Industries, Ltd. | 038-02606 | ESL3428 |
| methanol | Wako Pure Chemical Industries, Ltd. | 130-16585 | APF2160 |
| RK-163 | provided by RIKEN | | F-01 |
| HSPC | Lipoid | LIPOID S PC-3 Batch No. 525600-2190668-01/023 | |
| MPEG2000-DSPE, Na salt | Lipoid | LIPOID PE 18:0/18:0-PEG 2000 Batch No. 588200-2190092-01/030 | |
| Cholesterol | KANTO CHEMICAL CO., INC. | 07331-30 | 901B2144 |
| 9% sucrose aqueous solution | prepared by dissolving sucrose at 9% in ultrapure water, and passing through filter for filtration sterilization with pore size 0.22 µm | | |

### (Preparation method)

### · For safety test

HSPC, MPEG2000-DSPE, Na salt, and cholesterol were placed in one flask at 14.5 mg, 4.88 mg, and 4.88 mg, respectively, and dissolved in 10 mL of a solution of methanol:chloroform = 1:2 (v/v). 1.0 mL of the solution was mixed with 1.5 mL of a solution of RK-163 (100 µg/mL in methanol:chloroform = 1:2), and the mixture was dried by blowing nitrogen while heating, and then left standing in a desiccator. 10 mL of a 9% sucrose aqueous solution was added to the dried mixture, and the mixture was emulsified with a Teflon homogenizer and further sized with an extruder (heated to 60°C, polycarbonate membrane filter with a pore size of 0.2 µm, one sheet). The prepared liposome dispersion was further diluted 1000-fold with a 9% sucrose aqueous solution, filtered with a PVDF membrane filter for filtration sterilization with a pore size of 0.22 µm, placed in a glass bottle with a screw cap, and stored at 4°C. This solution was used as sample #8 (for safety test).

### · For main pharmacological test

1 mL of sample #8 (for safety test) was taken in another glass bottle, and 9 mL of 9% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #8 (for main pharmacological test).

### [Preparation of sample #9 (= RCAI-61-containing liposome: (#9))]

Using the reagents and ingredients shown in Table 11 and according to the following preparation method, sample #9 for safety test and main pharmacological test was prepared. Sample #9 was produced by the same preparation method as in sample #7 except that RCAI-61 was used instead of RK-163.

### (Composition of prepared sample)

### · For safety test

15 ng/mL RCAI-61, 220.8 ng/mL total lipid in 9% sucrose aqueous solution
lipid composition: HSPC 103 ng/mL, DOPS 79.7 ng/mL, cholesterol 38.1 ng/mL

### · For main pharmacological test

1.5 ng/mL RCAI-61, 22.08 ng/mL total lipid in 9% sucrose aqueous solution
lipid composition: HSPC 10.3 ng/mL, DOPS 7.97 ng/mL, cholesterol 3.81 ng/mL
particle size: 145.7 nm PDI: 0.06813

**[Table 11]**

| | Manufacturing company or preparation method | Cat No. | Lot No. |
|---|---|---|---|
| chloroform | Wako Pure Chemical Industries, Ltd. | 038-02606 | ESL3428 |
| methanol | Wako Pure Chemical Industries, Ltd. | 130-16585 | APF2160 |
| RCAI-61 | provided by RIKEN | | |
| HSPC | Lipoid | LIPOID S PC-3 Batch No. 525600-2190668-01/023 | |
| DOPS | NIPPON FINE CHEMICAL CO., LTD. | - | RGF-NF 231 |
| Cholesterol | KANTO CHEMICAL CO., INC. | 07331-30 | 901B2144 |
| 9% sucrose aqueous solution | prepared by dissolving sucrose at 9% in ultrapure water, and passing through filter for filtration sterilization with pore size 0.22 µm | | |

### (Preparation method)

### · For safety test

HSPC, DOPS, and cholesterol were placed in one flask at 10.3 mg, 7.97 mg, and 3.81 mg, respectively, and dissolved in 10 mL of a solution of methanol:chloroform = 1:2 (v/v). 1.0 mL of the solution was mixed with 1.5 mL of a solution of RCAI-61 (100 µg/mL in methanol:chloroform = 1:2), and the mixture was dried by blowing nitrogen while heating, and then left standing in a desiccator. 10 mL of a 9% sucrose aqueous solution was added to the dried mixture, and the mixture was emulsified with a Teflon homogenizer and further sized with an extruder (heated to 50°C, polycarbonate membrane filter with a pore size of 0.2 µm, one sheet). The prepared liposome dispersion was further diluted 1000-fold with a 9% sucrose aqueous solution, filtered with a PVDF membrane filter for filtration sterilization with a pore size of 0.22 µm, placed in a glass bottle with a screw cap, and stored at 4°C. This solution was used as sample #9 (for safety test).

### · For main pharmacological test

1 mL of sample #9 (for safety test) was taken in another glass bottle, and 9 mL of 9% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #9 (for main pharmacological test).

### [Preparation of sample #10 (= RCAI-137-containing liposome: (#10))]

Using the reagents and ingredients shown in Table 12 and according to the following preparation method, sample #10 for safety test and main pharmacological test was prepared. Sample #10 was produced by the same preparation method as in sample #7 except that RCAI-137 was used instead of RK-163.

### (Composition of prepared sample)

### · For safety test

15 ng/mL RCAI-137, 220.8 ng/mL total lipid in 9% sucrose aqueous solution
lipid composition: HSPC 103 ng/mL, DOPS 79.7 ng/mL, cholesterol 38.1 ng/mL

### · For main pharmacological test

1.5 ng/mL RCAI-137, 22.08 ng/mL total lipid in 9% sucrose aqueous solution
lipid composition: HSPC 10.3 ng/mL, DOPS 7.97 ng/mL, cholesterol 3.81 ng/mL
particle size: 145.7 nm PDI: 0.06813

**[Table 12]**

| | Manufacturing company or preparation method | Cat No. | Lot No. |
|---|---|---|---|
| chloroform | Wako Pure Chemical Industries, Ltd. | 038-02606 | ESL3428 |
| methanol | Wako Pure Chemical Industries, Ltd. | 130-16585 | APF2160 |
| RCAI-137 | provided by RIKEN | | |
| HSPC | Lipoid | LIPOID S PC-3 Batch No. 525600-2190668-01/023 | |
| DOPS | NIPPON FINE CHEMICAL CO., LTD. | | RGF-NF 231 |
| Cholesterol | KANTO CHEMICAL CO., INC. | 07331-30 | 901B2144 |
| 9% sucrose aqueous solution | prepared by dissolving sucrose at 9% in ultrapure water, and passing through filter for filtration sterilization with pore size 0.22 µm | | |

### (Preparation method)

### · For safety test

HSPC, DOPS, and cholesterol were placed in one flask at 10.3 mg, 7.97 mg, and 3.81 mg, respectively, and dissolved in 10 mL of a solution of methanol:chloroform = 1:2 (v/v). 1.0 mL of the solution was mixed with 1.5 mL of a solution of RCAI-137 (100 µg/mL in methanol:chloroform = 1:2), and the mixture was dried by blowing nitrogen while heating, and then left standing in a desiccator. 10 mL of a 9% sucrose aqueous solution was added to the dried mixture, and the mixture was emulsified with a Teflon homogenizer and further sized with an extruder (heated to 50°C, polycarbonate membrane filter with a pore size of 0.2 µm, one sheet). The prepared liposome dispersion was further diluted 1000-fold with a 9% sucrose aqueous solution, filtered with a PVDF membrane filter for filtration sterilization with a pore size of 0.22 µm, placed in a glass bottle with a screw cap, and stored at 4°C. This solution was used as sample #10 (for safety test).

### · For main pharmacological test

1 mL of sample #10 (for safety test) was taken in another glass bottle, and 9 mL of 9% sucrose aqueous solution was added and diluted by mixing. The liposome dispersion was filtered with a PVDF membrane filter for filtration sterilization with 0.22 µm pore size and stored at 4°C in a glass bottle with a screw cap. This solution was used as sample #10 (for main pharmacological test).

### [Experimental Example 1] Antitumor effect of NKT cell ligand (RK-163)-containing liposome

To examine the effectiveness of RK-163-containing liposomes with different liposome compositions (samples #2-#8 produced using the above-mentioned preparation method), the effectiveness in antitumor activity was examined.

In Experimental Example 1-1, 0.3 ng (converted to RK-163 amount) of RK-163-containing liposome (#2-#5) sample (for main pharmacological test) and, in Experimental Example 1-2, 0.3 ng (converted to RK-163 amount) of RK-163-containing liposome (#5-#8) sample (for main pharmacological test) and 1×10⁶ cells of B16 mouse melanoma cells (MO4) expressing the pseudo-cancer antigen OVA irradiated with X-rays (50 Gy) were administered to the tail vein of C57BL/6 mice (10 weeks old). Thereafter, 3×10⁵ cells of B16 mouse melanoma cells (MO4) were subcutaneously transplanted into the same mice. After transplantation, the tumor diameter was observed on days 7, 10, 14, and 17. All measurements were performed using digital calipers. Tumor volume (mm³) was calculated by major axis (mm) × minor axis (mm) × height (mm).

As a comparative example, the test was performed by the same method except that RK-163-containing CAPTISOL aqueous solution (sample #1 (for main pharmacological test)) was used instead of the RK-163-containing liposome. A group administered with the same amount of PBS as the RK-163-containing liposome was used as a control group.

The results are shown in Fig. 1.

As a result, by a comparison of RK-163-containing liposome: (#2-#5) (Experimental Example 1-1) with RK-163-containing liposome: (#5-#8) (Experimental Example 1-2), RK-163-containing liposome: (#7) and RK-163-containing liposome: (#8) showed significant antitumor effects.

### [Experimental Example 2] Antitumor activity (dose) and ED50 by NKT cell ligand (RK-163)-containing liposome

RK-163-containing liposome: (#7) (0.001 ng/25 g body weight to 1 ng/25 g body weight, converted to RK-163 amount, was administered) and MO4 cells expressing the pseudo-cancer antigen OVA irradiated with X-rays (50 Gy) were administered to the tail vein of C57BL/6 mice (10 weeks old). Thereafter, 3×10⁵ cells of B16 mouse melanoma cells (MO4) were subcutaneously transplanted into the same mice. After transplantation, the tumor diameter was observed on days 7, 11, 14, and 17. All measurements were performed using digital calipers. Tumor volume (mm³) was calculated by major axis (mm) × minor axis (mm) × height (mm). A group administered with the same amount of PBS instead of the RK-163-containing liposome: (#7) was used as a control.

The results are shown in Fig. 2.

As a result, RK-163-containing liposome: (#7) showed a strong antitumor effect and suppressed the proliferation of melanoma tumor cells in proportion to the amount of RK-163 (i.e., in a dose-dependent manner). The effective dose, converted to RK-163 amount, was 0.3 ng/25 g mouse body weight to 1 ng/25 g mouse body weight. The ED50 was 0.01393.

### [Experimental Example 3] Antitumor effect of NKT cell ligand (RCAI-61 or RCAI-137)-containing liposome

RK-163-containing liposome: (#7), RCAI-61-containing liposome: (#9) or RCAI-137-containing liposome: (#10) (0.003 ng/25 g body weight to 1 ng/25 g body weight, converted to the amount of RK-163, RCAI-61, or RCAI-137, was administered) and MO4 cells expressing the pseudo-cancer antigen OVA irradiated with X-rays (50 Gy) were administered to the vein of C57BL/6 mice (10 weeks old). Thereafter, 3×10⁵ cells of B16 mouse melanoma cells (MO4) were subcutaneously transplanted into the same mice. After transplantation, the tumor diameter was observed on days 7, 11, 14, and 17. All measurements were performed using digital calipers. Tumor volume (mm³) was calculated by major axis (mm) × minor axis (mm) × height (mm). A group administered with the same amount of PBS instead of the RK-163, RCAI-61, or RCAI-137-containing liposome was used as a control.

The results are shown in Fig. 3.

As a result, both the RCAI-61-containing liposome: (#9) and RCAI-137-containing liposome: (#10) showed a strong antitumor effect, like the RK-163-containing liposome: (#7) that showed the effectiveness in Experimental Example 1.

### [Experimental Example 4] Liver toxicity test of NKT cell ligand (RK-163)-containing liposome

A liver toxicity test (liver toxicity: ALT, AST, liver white spots) of RK-163-containing liposome: (#7) was performed using 10 times the effective amount (10 ng/mouse, 25 g body weight) of the antitumor effect shown in Fig. 2 (1 ng/mouse, 25 g body weight) as the results of Experimental Example 2. 10 ng of RK-163-containing liposome: (#7) was administered from the tail vein of C57BL/6 mice (10 weeks old) (5 mice per group), blood was collected on days 3, 7, and 17 after administration, and ALT and AST were measured by ELISA. In addition, liver was removed from the mice on day 17 after administration and the presence or absence of white spots on the liver was confirmed. Groups administered with the same amount of liposome (LIPO) not containing RK-163 or the same amount of PBS, instead of the RK-163-containing liposome: (#7), were used as control.

The results are shown in Fig. 4. As a result, even when the RK-163-containing liposome containing 10 times the effective amount for antitumor effect was administered, an increase in liver enzymes AST and ALT or white spots on the liver was not observed and the safety was confirmed.

The "standard value upper limit" in Fig. 4 was calculated statistically by CLEA Japan, Inc. from data of normal 10-week-old C57BL/6 mice, wherein ALT (GPT) was 34.4 IU/L and AST (GOT) was 77.4 IU/L. Therefore, values not more than the "standard value upper limit", 0-34.4 IU/L (ALT) and 0-77.4 IU/L (AST), are normal values.

### [Experimental Example 5-1] Activation of NKT cells outside tumor tissue by NKT cell ligand (RK-163)-containing liposome

An effective amount of RK-163-containing liposome: (#7) (1 ng/mouse, converted to RK-163 amount) and a pseudo-cancer antigen (OVA-pulsed C57BL/6J mouse splenocytes, 2×10⁷ cells) were administered from the tail vein of C57BL/6 mice (10 weeks old) (5 mice per group) to have the RK-163-containing liposome: (#7) taken up by antigen-presenting cells in the liver (e.g., Kupffer cell and the like), and NKT cells in the liver were activated by the RK-163 ligand antigen-presented on COld molecules. As a result, mouse NKT cells expressed the activation marker KLRG1 on the cell surface and produced IFN-γ, an adjuvant substance that activates other immune cell groups. A group administered with the same amount of PBS instead of the RK-163-containing liposome: (#7) was used as a control group.

The results are shown in Fig. 5-1 (Fig. 5-1 shows data three days after administration of RK-163-containing liposome: (#7)). NKT cells were activated by the RK-163-containing liposome, increased in number, and induced the expression of the activation marker KLRG1 and the production of IFN-γ.

### [Experimental Example 5-2] In vitro activation of human NKT cells by NKT cell ligand (RK-163)-containing liposome

The activation (IFN-γ production) of human NKT cells by RK-163-containing liposome: (#7) was examined.

After removing CD3⁺ cell groups, including NKT cells, from splenocytes of C57BL/6 mice by MACS, the cells were seeded at 5.0×10⁴ cells/well in a 96-well plate. An effective amount of RK-163-containing liposome: (#7) (1 ng, converted to RK-163 amount) was added and pulse cultured at 37°C for 24 hr. Thereafter, the culture supernatant was removed, and human NKT hybridoma cells (Raoult cells) were seeded at 5.0×10⁴ cells/well and cultured for an additional 24 hr at 37°C. The culture supernatant was collected and the amount of IFN-γ produced in the culture supernatant was measured using Human IFN-γ ELISA. A group administered with the same amount of the culture medium (medium) instead of the RK-163-containing liposome: (#7) was used as a control group.

The results are shown in Fig. 5-2.

As a result, the production of IFN-γ significantly increased in the RK-163-containing liposome: (#7) administration group. These results indicate that RK-163-containing liposome: (#7) pulsed on mouse antigen-presenting cells activates human NKT cells and produces IFN-γ.

These results demonstrate that (1) RK-163-containing liposome can activate human NKT cells, and (2) RK-163-containing liposome presented on mouse antigen-presenting cells can also activate human NKT cells, demonstrating that NKT cells can be activated across species.

### [Experimental Example 6] Activation of NKT cells outside tumor tissue by NKT cell ligand (RCAI-61 or RCAI-137)-containing liposome

RACI-61-containing liposome: (#9) or RCAI-137-containing liposome: (#10) (1 ng/mouse, converted to the amount of RCAI-61 or RCAI-137) and pseudo-cancer antigen OVA-pulsed splenocytes were administered from the tail vein of C57BL/6 mice to have the RACI-61-containing liposome: (#9) or RCAI-137-containing liposome: (#10) taken up by antigen-presenting cells in the liver (Kupffer cell and the like), and NKT cells in the liver were activated by the RCAI-61 or RCAI-137 ligand antigen-presented on CDld molecules.

The results are shown in Fig. 6.

As a result, administration of RACI-61-containing liposome: (#9) or RCAI-137-containing liposome: (#10) caused marked proliferation of NKT cells (Fig. 6, left diagram), expression of the activation marker KLRG1 on the cell surface, and production of IFN-γ, which is an adjuvant substance that activates other immune cell groups (Fig. 6, right diagram).

These results demonstrate that NKT cells artificially activated outside tumor tissue can infiltrate tumor tissue and activate immune cell groups within the tumor tissue.

### [Experimental Example 7] Activation of innate immune system · acquired immune system cell groups by NKT cell ligand (RK-163)-containing liposome-activated NKT cells

An effective amount of RK-163-containing liposome: (#7) (1 ng/mouse, converted to RK-163 amount) and OVA-pulsed splenocytes (2×10⁷ cells) as pseudo-cancer antigen were administered from the tail vein of C57BL/6 mice (10 weeks old) (5 mice per group) to activate NKT cells in the liver. The adjuvant action of NKT cell activation was analyzed for the influence on the innate immune system and acquired immune system cell groups, by using PD-1 expression as an index. A group administered with the same amount of PBS instead of the RK-163-containing liposome: (#7) was used as a control group.

The results are shown in Fig. 7.

As a result, after activation of NKT cells by RK-163-containing liposome: (#7) (see Experimental Example 5-1, Fig. 5-1), the acquired immune system CD4T, CD8T cells, and innate immune system NK cells were activated, and the expression of PD-1 as an activation index was confirmed along with an increase in the number of cells. Administration of RK-163 liposome activated NKT cells, thereby activating not only the acquired immune system CD4T cells and CD8T cells, but also the innate immune system NK cells.

### [Experimental Example 8] Activation of innate immune system · acquired immune system cell groups by NKT cell ligand (RCAI-61 or RCAI-137)-containing liposome-activated NKT cells

RACI-61-containing liposome: (#9) or RCAI-137-containing liposome: (#10) and pseudo-cancer antigen OVA-pulsed splenocytes were administered from the tail vein of C57BL/6 mice. In order to verify that the adjuvant action of the activated NKT cell activates the innate immune system and acquired immune system cell groups, the cell number and PD-1 expression were analyzed.

The results are shown in Fig. 8.

As a result, both the RACI-61-containing liposome: (#9) (Fig. 8, left diagram) and RCAI-137-containing liposome: (#10) (Fig. 8, right diagram) activated acquired immune system CD4T, CD8T cells, and innate immune system NK cell after activation of NKT cells, and the expression of PD-1 as an activation index was confirmed along with an increase in the number of cells.

### [Experimental Example 9] Infiltration into tumor tissue and activation of innate immune system (NK) · acquired immune system effector (CD8T) cell groups by NKT cell ligand (RK-163)-containing liposome-activated NKT cells

An effective amount of RK-163-containing liposome: (#7) (1 ng/mouse body weight, converted to RK-163 amount) and 1×10⁶ X-ray-irradiated MO4 cells (OVA-expressing B16 mouse melanoma cells) as pseudo-cancer antigen were administered from the tail vein of mice (C57BL/6J, 10 weeks old, 5 per group). Thereafter, 3×10⁵ MO4 tumor cells were transplanted subcutaneously, and immune cell analysis within the tumor was performed 17 days later. A group administered with the same amount of PBS instead of the RK-163-containing liposome: (#7) was used as a control group.

The results are shown in Fig. 9.

As a result, in the RK-163-containing liposome: (#7) administration group, the artificially activated NKT cells infiltrated the tumor tissue (Fig. 9, left, upper diagram), produced IFN-γ in the tumor tissue (Fig. 9, left, lower diagram), and activated the acquired immune system CD8T cells present in the tumor tissue and also the innate immune NK cells (Fig. 9, right diagram). On the other hand, in the PBS administration group, almost the same number of artificially non-activated NKT cells infiltrated into the tumor tissue (Fig. 9, left, upper diagram), but because they did not produce IFN-γ (Fig. 9, left, lower diagram), they did not activate the innate immune system or acquired immune group, and the expression of PD-1 as an activation marker was not observed (Fig. 9, right diagram). This shows that artificial activation of NKT cells is important.

NKT cells artificially activated outside the tumor tissue infiltrate into the tumor and remain activated within the tumor tissue. They continue to produce large amounts of IFN-γ, which activates other immune cell groups. As a result, in the RK-163-containing liposome: (#7) administration group, CD8T cells that attack antigen-expressing positive cancer cells, including mutated cancer cells, and NK cell groups that attack cancer cells that have lost antigen expression were activated. Expression of PD-1 as an index of activation, was confirmed in these immune cell groups, indicating the activation of immune cell groups within the immunodeficient tumor.

### [Experimental Example 10] Long-term immune memory induction · maintenance (30, 90 days later) by NKT cell ligand (RK-163)-containing liposome

C57BL/6 mice (10 weeks old) (5 mice per group) were simultaneously immunized by intravenous administration of RK-163-containing liposome: (#7) (1 ng/mouse body weight, converted to RK-163 amount) and 2×10⁷ spleen cells pulsed with a pseudo-cancer antigen (OVA). The mice were sacrificed 30 and 90 days after administration of the RK-163-containing liposome: (#7), and the spleen cells were used for analysis.

The results are shown in Fig. 10.

As a result, the proliferation of OVA-specific CD8T cell group and the formation of long-term immune memory CD8T cell group (significant detection of Tern and Tern fractions of OVA-specific CD8 cells) were confirmed by the administration of RK-163-containing liposome. Also, it was confirmed that long-term memory was formed and maintained 30 days and 90 days after the administration of RK-163-containing liposome: (#7). It was shown that administration of RK-163-containing liposome results in the formation of cancer-specific CD8T memory cells which are maintained for 30 days and 90 days.

### [Experimental Example 11] Induction and maintenance of long-term immune memory by NKT cell ligand (RCAI-61 or RCAI-137)-containing liposome (after 30 and 90 days)

C57BL/6 mice were immunized by intravenous administration of RCAI-61-containing liposome: (#9) or RCAI-137-containing liposome: (#10) and spleen cells pulsed with a pseudo-cancer antigen (OVA). The mice were sacrificed 30 days and 90 days after administration of RCAI-61-containing liposome: (#9) or RCAI-137-containing liposome: (#10), and the spleen cells were used for analysis.

The results are shown in Fig. 11.

As a result, both the proliferation of OVA-specific CD8T cell group and the formation of long-term immune memory CD8T cell group (significant detection of Tern and Tern fractions of OVA-specific CD8 cells) were confirmed after the administration of RCAI-61-containing liposome (Fig. 11, upper diagram) or RCAI-137-containing liposome (Fig. 11, lower diagram). Also, it was confirmed that long-term memory was formed and maintained 30 days and 90 days after the administration of RCAI-61-containing liposome (Fig. 11, upper diagram) or RCAI-137-containing liposome (Fig. 11, lower diagram).

### [Experimental Example 12] Antitumor effect after one year from establishment and maintenance of long-term immune memory by NKT cell ligand (RK-163)-containing liposome

C57BL/6 mice (10 weeks old) were simultaneously immunized with RK-163-containing liposome: (#7) and 2×10⁷ spleen cells pulsed with pseudo-cancer antigen (OVA) and left standing.

After lapse of one year, B16 mouse melanoma cells (MO4) (3×10⁵ cells) were subcutaneously transplanted into the long-term memory-induced C57BL/6 mice, and the tumor diameter was observed on days 7, 10, 14, and 17. All measurements were performed using digital calipers. Tumor volume (mm³) was calculated by major axis (mm) × minor axis (mm) × height (mm).

Using NKT cell-deficient NKT-KO mouse instead of C57BL/6 mouse, a similar experiment was performed. In addition, a group administered with the same amount of a liposome preparation not containing RK-163 (LIPO) instead of RK-163-containing liposome: (#7) was used as a control group.

The results are shown in Fig. 12.

As a result, in a group of C57BL/6 mice administered with the RK-163-containing liposome: (#7), when tumor cells were transplanted into individuals one year after a single administration of RK-163-containing liposome: (#7), a strong antitumor effect was exhibited and tumor growth was suppressed. In other words, a single administration of RK-163-containing liposome: (#7) induced immune memory for not less than one year and suppressed tumor growth. On the other hand, in the control group administered with the liposome not containing RK-163, and in the group of NKT cell-deficient NKT-KO mice administered with RK-163-containing liposome, the onset of cancer could not be suppressed, meaning that long-term memory could not be induced.

RK-163-containing liposome: (#7) afforded a strong antitumor effect even one year after administration, and it was confirmed that it has the effect of establishing long-term cancer memory, which is important for cancer treatment.

The LIPO administration group and NKT cell-deficient NKT-KO mice, as control groups, could not induce long-term memory, and as a result, could not suppress the onset of cancer. Therefore, it was verified that activated NKT cells are essential cells for long-term immune memory.

### [Industrial Applicability]

According to the present invention, an NKT cell ligand-containing preparation that can artificially activate NKT cells in a living body and is useful as an anti-malignant tumor agent and the like can be provided.

This application is based on patent application No. 2022-020306 filed in Japan, the contents of which are encompassed in full herein.

## Claims

1. A liposome composition comprising a glycolipid selected from the group consisting of a compound represented by the formula (I), a compound represented by the formula (II), and salts thereof:
the formula (I): 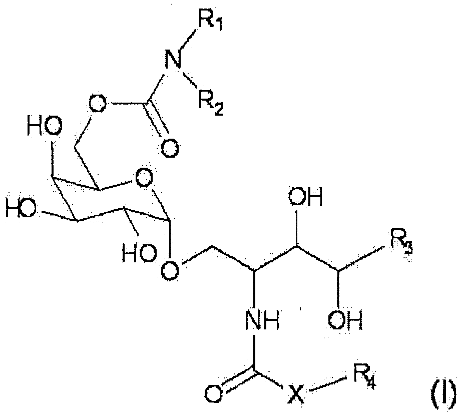
wherein X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30;
the formula (II): 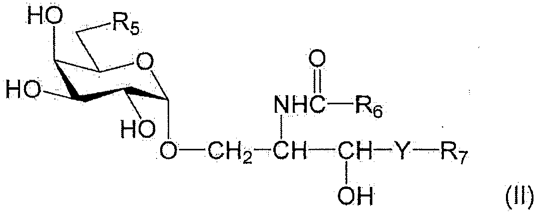
wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.

2. The composition according to claim 1, wherein the aforementioned glycolipid is a compound represented by the formula (I) or a salt thereof.

3. The composition according to claim 2, wherein the aforementioned glycolipid is a compound represented by the following formula: 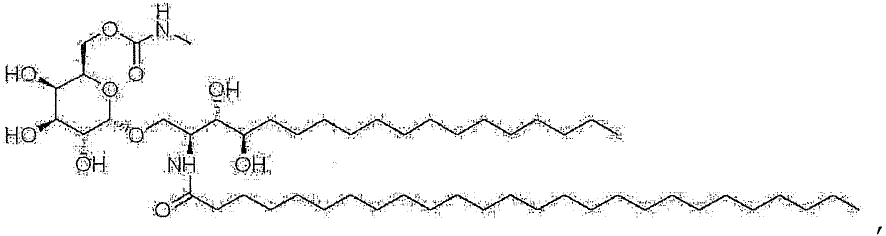 or a salt thereof.

4. The composition according to any one of claims 1 to 3, which is an anti-malignant tumor agent or an anti-infective agent.

5. The composition according to any one of claims 1 to 3, which is an NKT cell activator.

6. The composition according to any one of claims 1 to 3, which is an IFN-γ production inducer.

7. The composition according to any one of claims 1 to 3, which is an activator for innate immune system lymphocyte and acquired immune system lymphocyte.

8. The composition according to any one of claims 1 to 3, which is a long-term immune memory inducer.

9. The composition according to any one of claims 1 to 3, for delivering the aforementioned glycolipid to an antigen-presenting cell.

10. A method for delivering a glycolipid selected from the group consisting of a compound represented by the formula (I), a compound represented by the formula (II), and salts thereof to an antigen-presenting cell, comprising administering a liposome composition comprising the glycolipid to a target:
the formula (I): 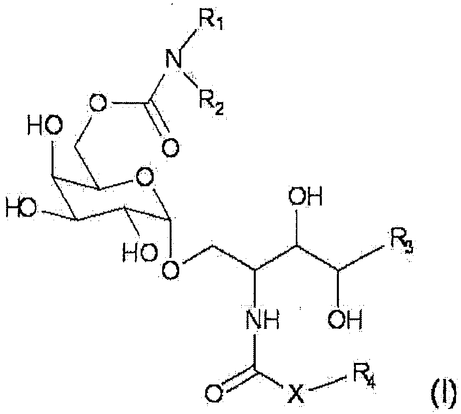
wherein X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30;
the formula (II): 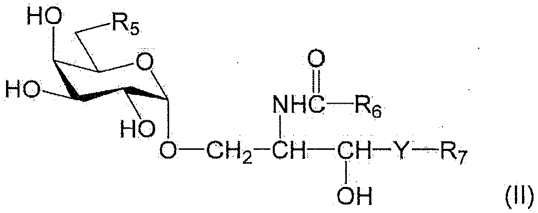 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.

11. A method for treating a malignant tumor or an infectious disease, comprising administering a liposome composition comprising a glycolipid selected from the group consisting of a compound represented by the formula (I), a compound represented by the formula (II), and salts thereof to a target:
the formula (I): 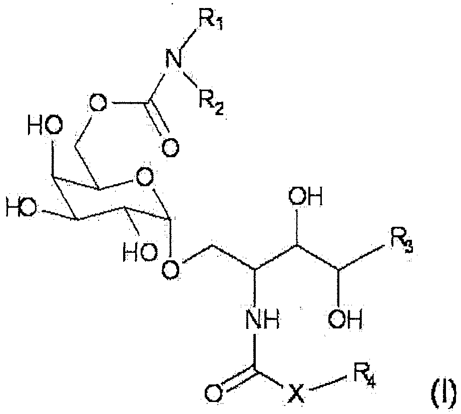
wherein X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30;
the formula (II): 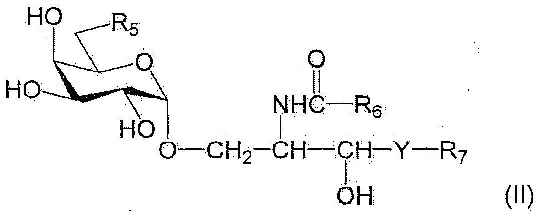 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.

12. A method for activating NKT cells in a target, comprising administering a liposome composition comprising a glycolipid selected from the group consisting of a compound represented by the formula (I), a compound represented by the formula (II), and salts thereof to the target:
the formula (I): 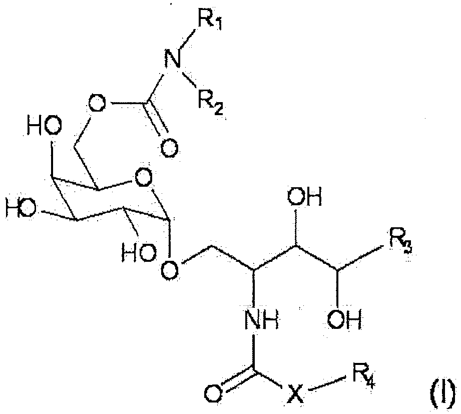
wherein X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30;
the formula (II): 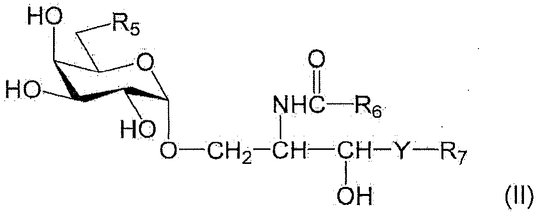 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.

13. A method for inducing IFN-γ production in a target, comprising administering a liposome composition comprising a glycolipid selected from the group consisting of a compound represented by the formula (I), a compound represented by the formula (II), and salts thereof to the target:
the formula (I): 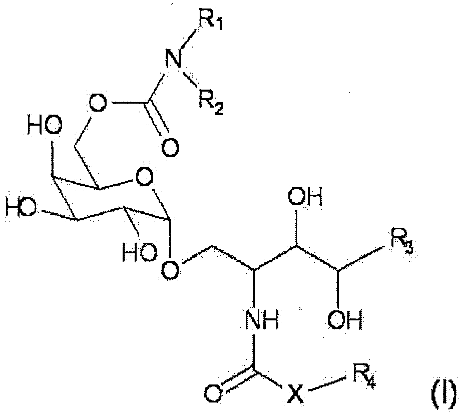
wherein X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30;
the formula (II): 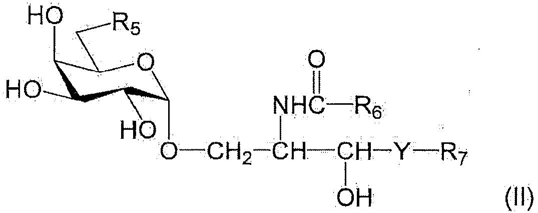 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.

14. A method for activating innate immune system lymphocytes and acquired immune system lymphocytes in a target, comprising administering a liposome composition comprising a glycolipid selected from the group consisting of a compound represented by the formula (I), a compound represented by the formula (II), and salts thereof to the target:
the formula (I): 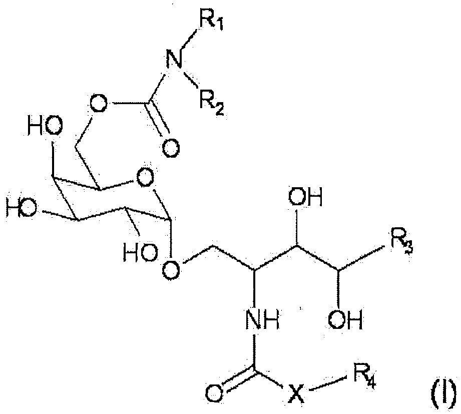
wherein X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30;
the formula (II): 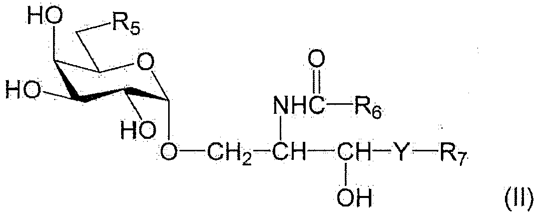 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.

15. A method for inducing a long-term immune memory in a target, comprising administering a liposome composition comprising a glycolipid selected from the group consisting of a compound represented by the formula (I), a compound represented by the formula (II), and salts thereof to the target:
the formula (I): 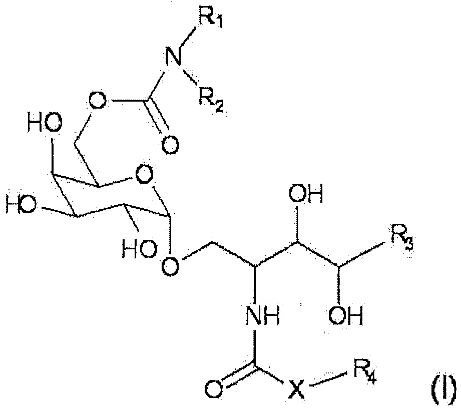
wherein X is an alkylene group or -NH-;
R₁ and R₂ are the same or different and each is a hydrogen atom, an alkyl group, a hydroxy group, an alkoxy group, or an aryl group optionally having substituent(s), R₁ and R₂ optionally form, together with the adjacent nitrogen atom, a 5- or 6-membered ring;
R₃ is a hydrocarbon group having a carbon number of 1 to 20; and
R₄ is a hydrocarbon group having a carbon number of 1 to 30;
the formula (II): 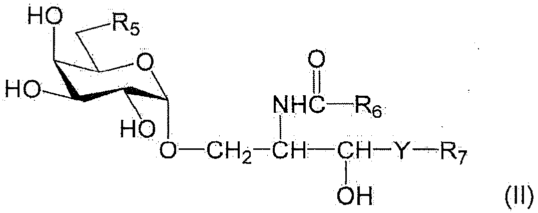 wherein R₅ is a hydrogen atom, an alkyl group having a carbon number of 1 to 7, an alkoxy group having a carbon number of 1 to 6 or a halogen atom, R₆ and R₇ are each independently a substituted or unsubstituted hydrocarbon group having a carbon number of 1 to 28, and Y is -CH₂-, -CH(OH)- or -CH=CH-.

16. The composition according to any one of claims 1 to 3, for use in delivering the aforementioned glycolipid to an antigen-presenting cell.

17. The composition according to any one of claims 1 to 3, for use in treating a malignant tumor or an infectious disease.

18. The composition according to any one of claims 1 to 3, for use in activating an NKT cell.

19. The composition according to any one of claims 1 to 3, for use in inducing IFN-γ production.

20. The composition according to any one of claims 1 to 3, for use in activating an innate immune system lymphocyte and an acquired immune system lymphocyte.

21. The composition according to any one of claims 1 to 3, for use in inducing a long-term immune memory.

22. The composition according to any one of claims 1 to 3, for use as a medicament.
